# EUROPEAN PATENT APPLICATION

(11) **EP 4 595 737 A1**
(43) Date of publication of application: **06.08.2025**
(21) Application number: 25155261.8
(22) Date of filing: 31.01.2025
(51) Int. Cl.: A01C 7/06, A01C 23/00, A01C 21/00, A01C 7/20

(54) **SYSTEM AND METHOD FOR MOISTURE DRIVEN MATERIAL APPLICATION**

(30) Priority: 31.01.2024 US 202463627556 P; 29.01.2025 US 202519040560
(71) Applicant: Deere & Company, Moline, IL 61265 (US)
(72) Inventor: HALBROOK, Michael L, Thibodaux, 70301 (US); YOUNG, Steven C, Urbandale, 50322 (US); TOSTENSON, Mary L, Urbandale, 50322 (US); BYRD, Seth A, Moline, 61265 (US); MINOR, Joshua M, Moline, 61265 (US)
(74) Representative: Reddie & Grose LLP

(57) **Abstract**

A planting machine (100) includes a processing system (266, 268), a moisture measurement device (115) in communication with the processing system (266, 268), a seed delivery system (166, 120) in communication with the processing system (266, 268), and a material (343) dispersion system (113, 166, 321, 124, 120, 180, 252, 256, 266, 268, 270, 278, 366, 368, 27, 29, 833, 834, 844) in communication with the processing system (266, 268). The processing system (266, 268) is configured to receive one (71, 863) or more soil (138) moisture measurement signals (304, 322) from the soil moisture measurement device (115), determine one (71, 863) or more material (343) dispersion parameters based at least in part on a moisture measurement signal (304), and transmit a control signal (304) to one or more of the seed delivery system (166, 120) and the material (343) dispersion system (113, 166, 321, 124, 120, 180, 252, 256, 266, 268, 270, 278, 366, 368, 27, 29, 833, 834, 844) to disburse a desired amount of material (343) with a commodity based at least in part on the determined material (343) dispersion parameters

## Description

### CROSS-REFERENCE TO RELATED APPLICATION

This application claims the benefit of U.S. Provisional Application No. 63/627556, filed January 31, 2024, which is hereby incorporated by reference in its entirety.

### FIELD OF THE DESCRIPTION

The present description relates to agricultural machines. More specifically, the present description relates to controlling application of material to a field, using an agricultural machine.

### BACKGROUND

There is a wide variety of different types of agricultural machines that apply material to an agricultural field. Some such agricultural machines include sprayers, tillage machines with side dressing bars, air seeders, and planters that have row units.

As one example, a row unit is often mounted to a planter with a plurality other row units. The planter is often towed by a tractor over soil where seed is planted in the soil, using the row units. The row units on the planter follow the ground profile by using a combination of a down force assembly that imparts a down force to the row unit to push disk openers into the ground and gauge wheels to set depth of penetration of the disk openers.

Row units can also be used to apply material to the field (e.g., fertilizer to the soil, to a seed, etc.) over which they are traveling. In some scenarios, each row unit has a valve that is coupled between a source of material to be applied, and an application assembly. As the valve is actuated, the material passes through the valve, from the source to the application assembly, and is applied to the field.

Many current systems apply the material in a substantially continuous way. For instance, where the application machine is applying a liquid fertilizer, it actuates the valve to apply a substantially continuous strip of the liquid fertilizer. The same is true of materials that provide other liquid substances, or granular substances, as examples. Many current systems apply the material uniformly in ground surfaces as material is applied to the field. For instance, in examples where the application machine is applying fertilizer or water, the planting machine applies fertilizer or water uniformly to ground surfaces of various compositions.

The discussion above is merely provided for general background information and is not intended to be used as an aid in determining the scope of the claimed subject matter.

### SUMMARY

In an illustrative example a planting machine includes a processing system, a moisture measurement device in communication with the processing system, a seed delivery system in communication with the processing system, and a material dispersion system in communication with the processing system. The processing system is configured to receive one or more soil moisture measurement signals from the soil moisture measurement device, determine one or more material dispersion parameters based at least in part on a moisture measurement signal, and transmit a control signal to one or more of the seed delivery system and the material dispersion system to disburse a desired amount of material with a commodity based at least in part on the determined material dispersion parameters.

In some examples, the planting machine includes one or more material dispersion parameters which may include whether to disperse the material onto the commodity or adjacent the commodity.

In some examples, the one or more material dispersion parameters includes an amount of material to disburse.

In some examples, the processing system is further configured to receive soil-type data. The one or more material dispersion parameters is based at least in part on the soil-type data.

In some examples, the processing system of the planting machine may be configured to determine a position of the planting machine determine associated soil parameters, and determine soil identification data based at least in part on the position of the planting machine and the associated soil parameters.

In some examples, the processing system of the planting machine is configured to determine one or more material dispersion parameters based at least in part on soil-type data.

In some examples, the planting machine processing system is configured to communicate moisture measurements from the moisture measurement device to a central server. In some examples, the moisture measurement device includes non-contact moisture measurement device.

In some examples, the moisture measurement device includes an electromagnetic wave-based moisture measurement device.

In some examples, the moisture measurement device includes an insertable moisture measurement device.

An illustrative example of a method of controlling a planting machine includes receiving one or more soil moisture measurement signals from the soil moisture measurement device, determining one or more material dispersion parameters based at least in part on one or more of the moisture measurement signals; and transmitting a control signal to the material dispersion system to disburse a desired amount of material with the commodity based at least in part on the determined amount of material.

In some examples, one or more material dispersion parameters includes, whether to disburse the material directly onto the commodity or adjacent the commodity.

In some examples, the one or more material dispersion parameters includes an amount of material to disburse.

In some examples, the method includes, receiving soil-composition data. The one or more material dispersion parameters is based at least in part on the soil-type data.

In some examples, the soil-composition data includes pre-measured data.

In some examples, the method includes determining a position of the planting machine, determining associated soil parameters, and determining soil identification data based at least in part on the position of the planting machine and the associated soil parameters.

In some examples, the position of the planting machine is based at least in part on global positioning data.

In some examples, the method includes determining one or more material dispersion parameters based at least in part on soil identification data.

In some examples, the method includes communicating soil identification data to a central server.

In some examples, one or more soil moisture measurement signals are based at least in part on electromagnetic wave measurement data.

### BRIEF DESCRIPTION OF THE DRAWINGS

FIG. 1 is a top view of one example of a planting machine, shown in a partial pictorial and partial schematic form.
FIG. 2 is a side view showing one example of a row unit of the planting machine illustrated in FIG. 1.
FIG. 2A is a view of an application unit.
FIG. 3 is a side view showing another example of a row unit of the planting machine illustrated in FIG. 1.
FIG. 4A is a side view showing another example of a row unit of the planting machine illustrated in FIG. 1.
FIG. 4B is a side view showing another example of a row unit of the planting machine illustrated in FIG. 1 and including a moisture sensor.
FIG. 5 is a perspective view of a portion of a seed metering system.
FIG. 6 shows an example of a seed delivery system that can be used with a seed metering system.
FIG. 7 shows another example of a delivery system that can be used with a seed metering system.
FIG. 8 is a block diagram showing one example of a material application control system.
FIG. 9A is a flow diagram illustrating one example of the operation of the material application control system shown in FIG. 8.
FIG. 9B is a flow diagram illustrating an example of a portion of the operation of the material application control system shown in FIG. 8.
FIG. 10 is a block diagram showing one example of an event driven processing system.
FIG. 11 is a flow diagram showing example of the operation of event driven processing.
   distance generation system.
FIG. 12 is a flow diagram showing one example of the operation of a time delay generation system.
   system.
FIG. 13 shows one example of the architecture illustrated in FIG. 1, deployed in a remote server environment.
FIGS. 14-15 show examples of mobile devices that can be used as operator interface mechanisms in the architectures shown in the previous Figures.
FIG. 16 is a block diagram showing one example of a computing environment that can be used in the architectures shown in the previous Figures.

### DETAILED DESCRIPTION

As discussed above, many current systems apply material to a field in a relatively continuous way. This can result in wasted material. For instance, some material that is applied at certain locations between seeds or plants in a field may be unnecessary. This can result in lower productivity and lower efficiency. This problem can be exacerbated in instances where the material is applied at a relatively high rate, such as in the case of high-rate fertilizer application. Additionally uniform application of material may not accommodate varying properties of ground materials in a field. Many current systems apply material to a field using uniform moisture and fertilizer additives irrespective of changing moisture levels in ground material throughout a planting operation. This can cause insufficient or excessive moisture or fertilizer during some or all of a planting operation.

The present description thus proceeds with respect to a system that identifies moisture level in a ground material during a planting operation and adjusts moisture and/or fertilizer dispensation based at least in part on the measured moisture level of the ground material. The system can detect the moisture level in the ground surface by processing measurement signals taken by a non-contact or a contact-based moisture sensor thus proceeds with respect to a system that identifies a moisture level in one or more ground materials and controllably dispenses or applies material (e.g., fertilizer, water, commodity, etc.) based at least in part on the measured moisture level in one or more locations (and/or positions) in a field. Additionally, the system can adjust the dispensation of the material as different moisture levels are detected during a seeding operation or a plurality of seeding operations. In some examples, a moisture map may be generated based on the moisture measurements taken at a plurality of locations. In some examples, additional data such as moisture differential (e.g., target vs. actual moisture) may be used to form the moisture map as well.

Further, the present description thus proceeds with respect to a system that identifies a specific location, e.g., a seed location, and controllably dispenses or applies material, based upon the seed location (and/or position) in a field. The system can do this by sensing seeds, as they are planted in the soil, and then calculating a time when an application valve or actuator, e.g., a pump, should be actuated to apply the material, based upon the location of the valve or actuator relative to the location of the seed.

The present description further proceeds with respect to the examples being deployed on a row unit of a planter. They could just as easily be deployed on a sprayer, an air seeder, a tillage machine with a side-dress bar, or other piece of agricultural equipment that is used to apply a material.

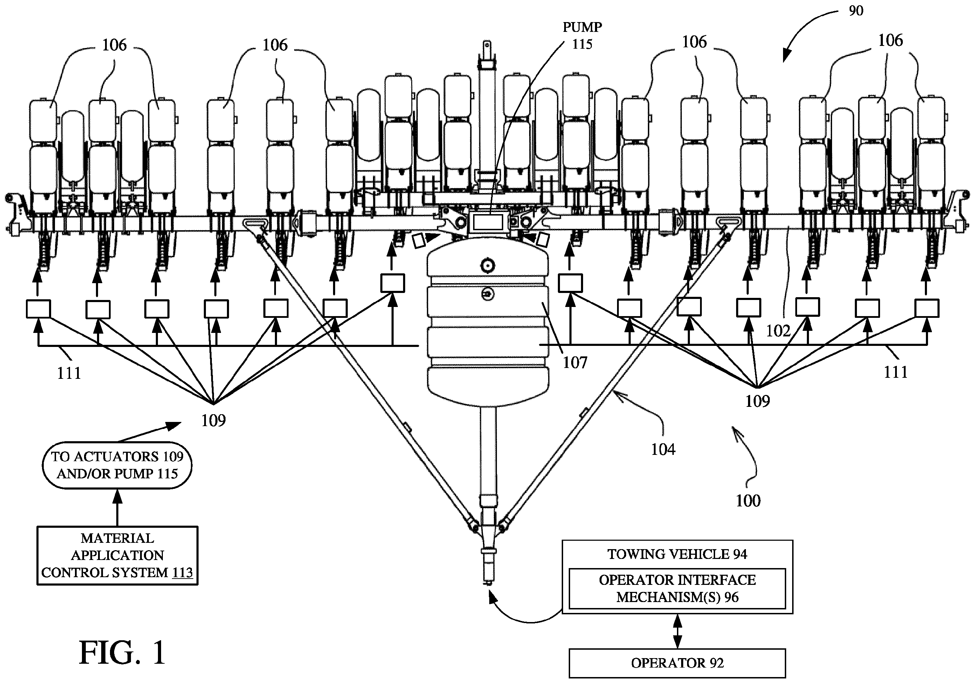

FIG. 1 is a partial pictorial, partial schematic top view of one example of an architecture 90 that includes agricultural planting machine 100, towing vehicle 94, that is operated by operator 92, and material application control system 113, which can be on one or more individual parts of machine 100, centrally located on machine 100, or on towing vehicle 94. Operator 92 can illustratively interact with operator interface mechanisms 96 to manipulate and control vehicle 94, system 113, and some or all portions of machine 100.

Machine 100 is a row crop planting machine that illustratively includes a toolbar 102 that is part of a frame 104. FIG. 1 also shows that a plurality of planting row units 106 are mounted to the toolbar 102. Machine 100 can be towed behind towing vehicle 94, such as a tractor. FIG. 1 shows that material can be stored in a tank 107 and pumped through a supply line 111 so the material can be dispensed in or near the rows being planted. In one example, a set of devices (e.g., actuators) 109 is provided to perform this operation. For instance, actuators 109 can be individual pumps that service individual row units 106 and that pump material from tank 107 through supply line 111 so it can be dispensed on the field. In such an example, material application control system 113 controls the pumps 109. In another example, actuators 109 are valves and one or more pumps 115 pump the material from tank 107 to valves 109 through supply line 111. In such an example, material application control system 113 controls valves 109 by generating valve or actuator control signals, e.g., on a per-seed basis, as described below. The control signal for each valve or actuator can, in one example, be a pulse width modulated control signal. The flow rate through the corresponding valve 109 can be based on the duty cycle of the control signal (which controls the amount of time the valve is open and closed). It can be based on multiple duty cycles of multiple valves or based on other criteria. Further, the material can be applied in varying rates on a per-seed or per-plant basis. For example, fertilizer may be applied at one rate when it is being applied at a location spaced from a seed location and at a second, higher, rate when it is being applied closer to the seed location. These are examples only.

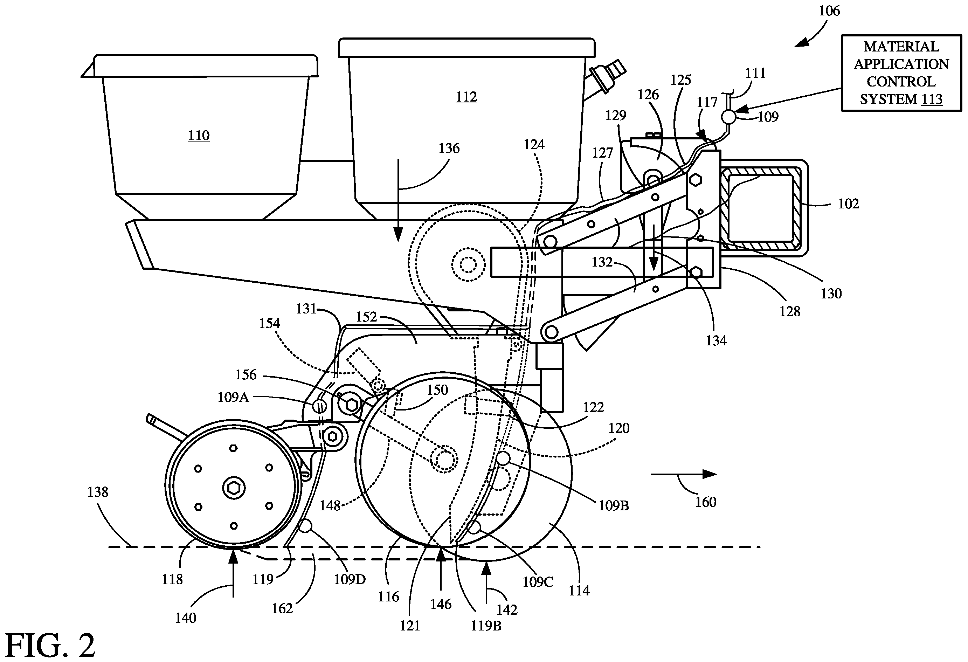

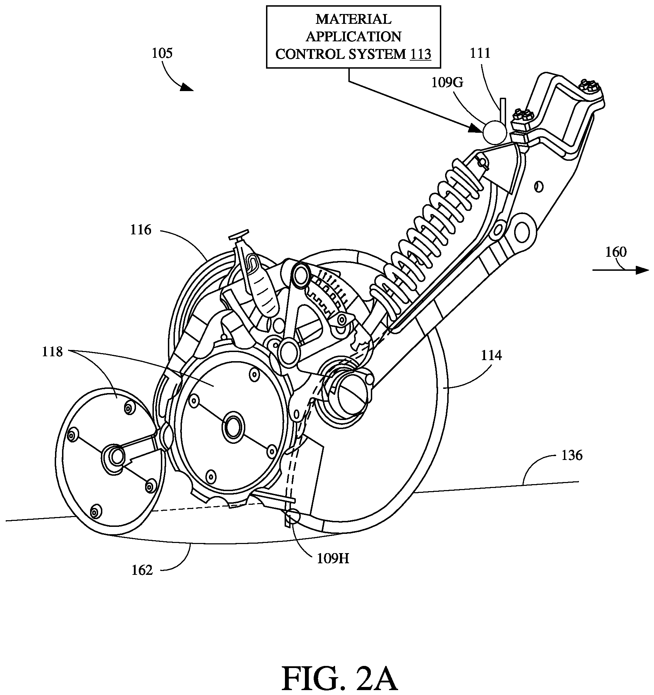

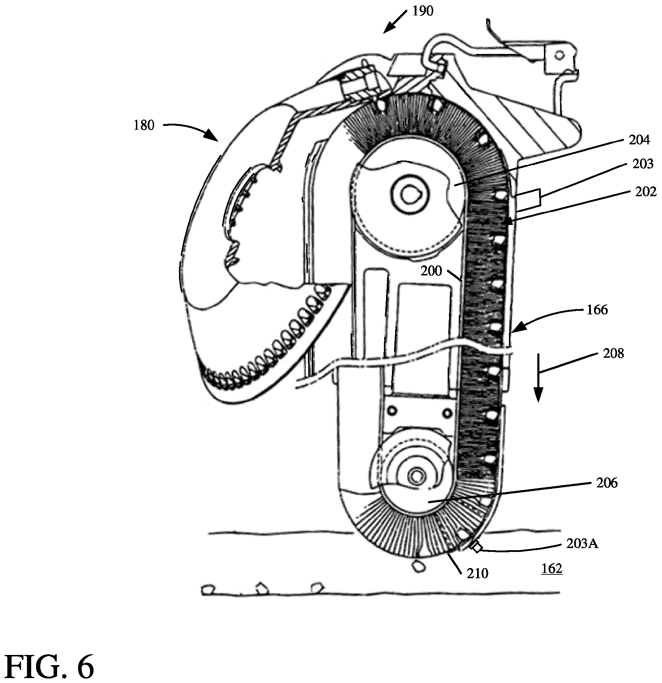

FIG. 2 is a side view of one example of a row unit 106, with actuator 109 and system 113 shown as well. Actuator 109 is shown in five possible locations labeled as 109, 109A, 109B, 109C and 109D. Row unit 106 illustratively includes a chemical tank 110 and a seed storage tank 112. It also illustratively includes one or more disc openers 114, a set of gauge wheels 116, and a set of closing wheels 118. Seeds from tank 112 are fed into a seed meter 124, e.g., by gravity or from a centralized commodity distribution system (e.g., exploiting pneumatic commodity distribution to each row unit). The seed meter 124 controls the rate at which seeds are dropped into a seed tube 120 or other seed delivery system, such as a brush belt or flighted belt (shown in FIGS. 6-7, respectively), from seed storage tank 112. The seeds can be sensed by a seed sensor 122.

In the example shown in FIG. 2, liquid material is passed, e.g., pumped or otherwise forced, through supply line 111 to an inlet end of actuator 109. Actuator 109 is controlled by control system 113 to allow the liquid to pass from the inlet end of actuator 109 to an outlet end.

As liquid passes through actuator 109, it travels through an application assembly 117 from a proximal end (which is attached to an outlet end of actuator 109) to a distal tip (or application tip) 119, where the liquid is discharged into a trench, or proximate a trench or furrow 162, opened by disc opener 114 (as is described in more detail below).

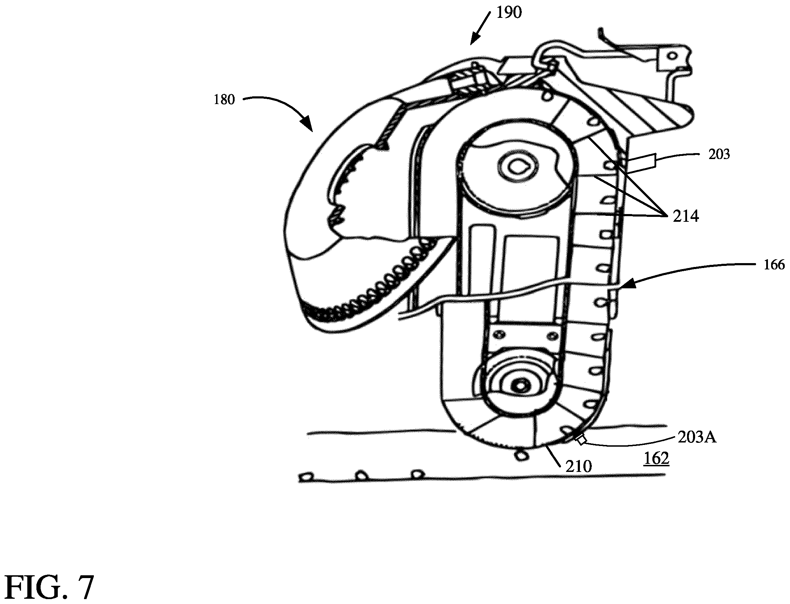

Some parts of row unit 106 will now be discussed in more detail. First, it will be noted that there are different types of seed meters 124, and the one that is shown is shown for the sake of example only and is described in greater detail below. However, in one example, each row unit 106 need not have its own seed meter. Instead, metering or other singulation or seed dividing techniques can be performed at a central location, for groups of row units 106. The metering systems can include finger pick-up discs and/or vacuum meters (e.g., having rotatable discs, rotatable concave or bowl-shaped devices), among others. The seed delivery system can be a gravity drop system (such as seed tube 120 shown in FIG. 2) in which seeds are dropped through the seed tube 120 and fall (via gravitational force) through the seed tube and out the outlet end 121 into the seed trench 162. Other types of seed delivery systems may be or may include assistive systems, in that they do not simply rely on gravity to move the seed from the metering system into the ground. Instead, such assistive systems actively assist the seeds in moving from the meter to a lower opening, where they exit or are deposited into the ground or trench. These can be systems that physically capture the seed and move it from the meter to the outlet end of the seed delivery system or they can be pneumatic systems that pump air through the seed tube to assist movement of the seed. The air velocity can be controlled to control the speed at which the seed moves through the delivery system. Some examples of assistive systems are described in greater detail below with respect to FIGS. 6 and 7.

A downforce actuator 126 is mounted on a coupling assembly 128 that couples row unit 106 to toolbar 102. Actuator 126 can be a hydraulic actuator, a pneumatic actuator, a spring-based mechanical actuator or a wide variety of other actuators. In the example shown in FIG. 2, a rod 130 is coupled to a parallel linkage 132 and is used to exert an additional downforce (in the direction indicated by arrow 134) on row unit 106. The total downforce (which includes the force indicated by arrow 134 exerted by actuator 126, plus the force due to gravity acting on row unit 106, and indicated by arrow 136) is offset by upwardly directed forces acting on closing wheels 118 (from ground 138 and indicated by arrow 140) and disc opener 114 (again from ground 138 and indicated by arrow 142). The remaining force (the sum of the force vectors indicated by arrows 134 and 136, minus the force indicated by arrows 140 and 142) and the force on any other ground engaging component on the row unit (not shown), is the differential force indicated by arrow 146. The differential force may also be referred to herein as the downforce margin. The force indicated by arrow 146 acts on the gauge wheels 116. This load can be sensed by a gauge wheel load sensor, which may be located anywhere on row unit 106 where it can sense that load. The gauge wheel load sensor can also be placed where it may not sense the load directly, but a characteristic indicative of that load. For example, it can be disposed near a set of gauge wheel control arms (or gauge wheel arm) 148 that movably mount gauge wheels 116 to shank 152 and control an offset between gauge wheels 116 and the discs in double disc opener 114, to control planting depth.

Arms (or gauge wheel arms) 148 illustratively abut against a mechanical stop (or arm contact member-or wedge) 150. The position of mechanical stop 150 relative to shank 152 can be set by a planting depth actuator assembly 154. Control arms 148 illustratively pivot around pivot point 156 so that, as planting depth actuator assembly 154 actuates to change the position of mechanical stop 150, the relative position of gauge wheels 116, relative to the double disc opener 114, changes, to change the depth at which seeds are planted.

In operation, row unit 106 travels generally in the direction indicated by arrow 160. The double disc opener 114 opens a furrow 162 in the soil 138, and the depth of the furrow 162 is set by planting depth actuator assembly 154, which, itself, controls the offset between the lowest parts of gauge wheels 116 and disc opener 114. Seeds are dropped through seed tube 120, into the furrow 162 and closing wheels 118 close the furrow 162, e.g., push soil back into the furrow 162.

As the seeds are dropped through seed tube 120, they can be sensed by seed sensor 122. Some examples of seed sensor 122 are described in greater detail below. Some examples of seed sensor 122 may include an optical or reflective sensor, which includes a radiation transmitter component and a receiver component. The transmitter component emits electro-magnetic radiation and the receiver component then detects the radiation and generates a signal indicative of the presence or absence of a seed adjacent the sensors. In another example, row unit 106 may be provided with a seed firmer that is positioned to travel through the furrow 162, after seeds are placed in furrow 162, to firm the seeds in place. A seed sensor can be placed on the seed firmer and generate a sensor signal indicative of a seed. Again, some examples of seed sensors are described in greater detail below.

The present description proceeds with respect to the seed sensor being located to sense a seed passing it in seed tube 120, but this is for the sake of example only. Material application control system 113 illustratively receives a signal from seed sensor 122, indicating that a seed is passing sensor 122 in seed tube 120. It then determines when to actuate actuator 109 so that material being applied through application assembly 117 (and out distal tip 119 of application assembly 117) will be applied at a desired location relative to the seed in trench or furrow 162. This is all described in greater detail herein as well. One brief example will be described now, by way of overview.

Material application control system 113 illustratively is programmed with, or detects a distance, e.g., a longitudinal distance, that the distal tip 119 is from the exit end 121 of seed tube 120. It also illustratively senses, or is provided (e.g., by another component, such as a GPS unit or a tractor, etc.), the ground speed of row unit 106. As the row units 106 on an implement being towed by a prime mover (e.g., a tractor) may move faster or slower than the tractor during turns, particularly as the width of the implement increases, the material application control system 113 may sense or be provided the ground speed of each row unit 106 of the implement. By way of example, the material application control system 113 may sense or be provided with information when the implement is turning right indicating that the rightmost row unit 106 is travelling slower, i.e., has a lower ground speed, than the leftmost row unit 106. Further, the material application control system 113 detects, is provided, or is programmed with, system data indicating the responsiveness of actuator 109 under certain conditions (such as under certain temperature conditions, certain humidity conditions, certain elevations, when spraying a certain type of fluid, etc.) and it also detects, is provided, or programmed with one or more properties of the material being applied through actuator 109 (as this may affect the speed at which actuator 109 responds, the time it takes for the material to travel through application assembly 117 to the distal tip 119 and be applied to furrow 162, etc.). Further, material application control system 113 illustratively detects (or is provided with a sensor signal indicative of) the forward speed of row unit 106 in the direction generally indicated by arrow 160.

With this type of information, once system 113 receives a seed sensor signal indicating that a seed is passing sensor 122 in seed tube 120, system 113 determines the amount of time it will take for the seed to drop through the outlet end of seed tube 121 and into furrow 162 to reside at its final seed location and position in furrow 162. It then determines when tip 119 will be in a desired location relative to that final seed location and it actuates valve 109 to apply the material at the desired location. By way of example, it may be that some material is to be applied directly on the seed. In that case, system 113 times the actuation of actuator 109 so that the applied material will be applied at the seed location. In another example, it may be desirable to apply some material at the seed location and also a predetermined distance on either side of the seed location. In that case, system 113 controls the signal used to control actuator 109 so that the material is applied in the desired fashion. In other examples, it may be that the material is to be applied at a location between seeds in furrow 162. By way of example, relatively high nitrogen fertilizer may be most desirably applied between seeds, instead of directly on the seed. In that case, system 113 has illustratively been programmed with the desired location of the applied material, relative to seed location, so that it can determine when to actuate actuator 109 in order to apply the material between seeds. Further, as discussed above, actuator 109 can be actuated to dispense material at a varying rate. It can dispense more material on the seed location and less at locations spaced from the seed location, or vice versa, or according to other patterns.

It will be noted that a wide variety of different configurations are contemplated herein. For instance, in one example, FIG. 2 shows that actuator 109 may be placed closer to the distal tip 119 (such as indicated by actuator 109A and 109C). In this way, there is less uncertainty as to how long it will take the material to travel from the actuator 109A and 109C to the distal tip 119. In yet another example, the valve is disposed at a different location (such as on seed tube 120) as indicated by actuator 109B and 109D. In those scenarios, again, actuator 109B and 109D are closer to the distal tip 119B and the material may be applied before and/or after the seed drops into furrow 162. For instance, when seed sensor 120 detects a seed, system 113 may be able to actuate valve 109B or 109D to apply material to furrow 162, before the seed exits the exit end 121 of seed tube 120. However, by the time the seed drops through distal end 121 of seed tube 120, the final seed location may be directly on the applied material. In yet another example, system 113 can control actuator 109B or 109D so that it applies material, but then stops applying it before the seed exits distal end 121. In that case, the material may be applied at a location behind the seed in furrow 162, relative to the direction indicated by arrow 160. This actuation timing enables the material to be applied between seeds, on seeds, or elsewhere. All of these and other configurations are contemplated herein.

FIG. 2A is a side perspective view of an applicator unit 105. Some items are similar to those shown in FIG. 2 and they are similarly numbered. Briefly, in operation, applicator unit 105 attaches to a side-dress bar that is towed behind a towing vehicle 94, so unit 105 travels between rows (if the rows are already planted). However, instead of planting seeds, it simply applies material at a location between rows of seeds (or, if the seeds are not yet planted, between locations where the rows will be, after planting). When traveling in the direction indicated by arrow, disc opener 114 (in this example, it is a single disc opener) opens furrow 162 in the ground 136, at a depth set by gauge wheel 116. When actuator 109 is actuated, material is applied in the furrow 162 and closing wheels 118 then close the furrow 162.

As unit 105 moves, material application control system 113 controls actuator 109 to dispense material. This can be done relative to seed or plant locations, if they are sensed or are already known or have been estimated. It can also be done before the seed or plant locations are known. In this latter scenario, the locations where the material is applied can be stored so that seeds can be planted later, relative to the locations of the material that has been already dispensed.

FIG. 2A shows that actuator 109 can be mounted to one of a plurality of different positions on unit 105. Two of the positions are shown at 109G and 109H. These are examples and the actuator 109 can be located elsewhere as well. Similarly, multiple actuators can be disposed on unit 105 to dispense multiple different materials or to dispense it in a more rapid or more voluminous way than is done with only one actuator 109.

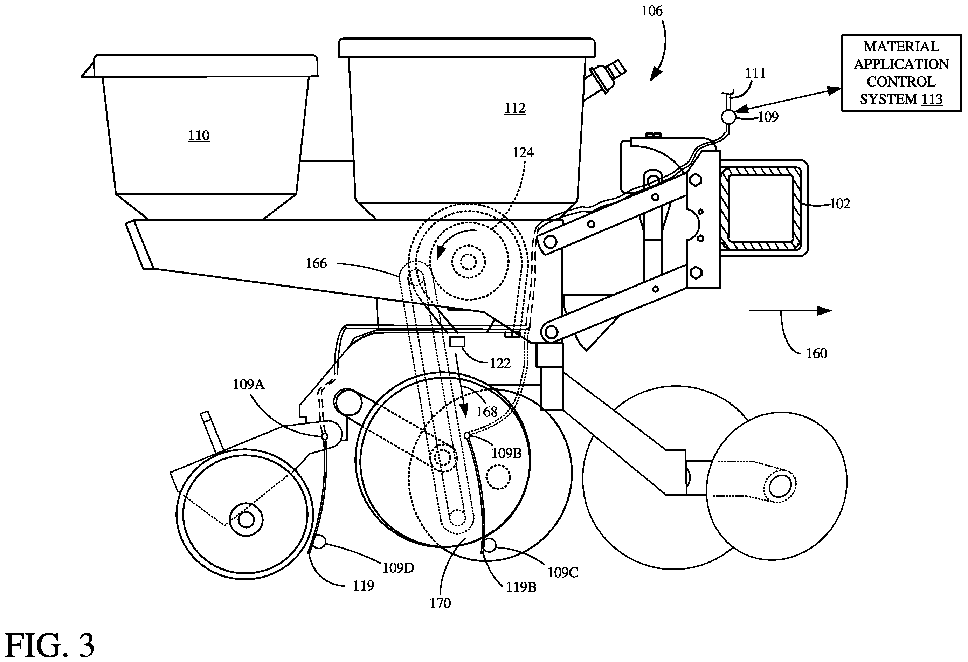

FIG. 3 is similar to FIG. 2, and similar items are similarly numbered. However, instead of the seed delivery system being a seed tube 120, which relies on gravity to move the seed to the furrow 162, the seed delivery system shown in FIG. 3 is an assistive seed delivery system 166. Assistive seed delivery system 166 also illustratively has a seed sensor 122 disposed therein. Assistive seed delivery system 166 captures the seeds as they leave seed meter 124 and moves them in the direction indicated by arrow 168 toward furrow 162. System 166 has an outlet end 170 where the seeds exit assistive system 166, into furrow 162, where they again reach their final resting location.

In such a system, material application control system 113 considers the speed at which delivery system 166 moves the seed from seed sensor 122 to the exit end 170. It also illustratively considers the speed at which the seed moves from the exit end 170 into furrow 162. For instance, in one example the seed simply drops from exit end 170 into furrow 162 under the force of gravity. In another example, however, the seed can be ejected from delivery system 166 at a greater or lesser speed than that which would be reached under the force of gravity. Similarly, it may be that the seed drops straight downward into furrow 162 from the outlet end 170. In another example, however, it may be that the seed is propelled slightly rearwardly from the outlet end 170, to accommodate for the forward motion of the row unit 106, so that the travel path of the seed is more vertical and so the seed rolls less once it reaches the furrow. Further, the seed can be ejected rearwardly and trapped against the ground by a trailing member (such as a pinch wheel) which functions to stop any rearward movement of the seed, after ejection, and to force the seed into firm engagement with the ground. Again, FIG. 3 also shows that valve 109 can be placed at any of a wide variety of different locations, some of which are illustrated by values 109A, 109B, 109C and 109D. There can be a more than one seed sensor, seed sensors of different types, different locations for seed sensors, etc.

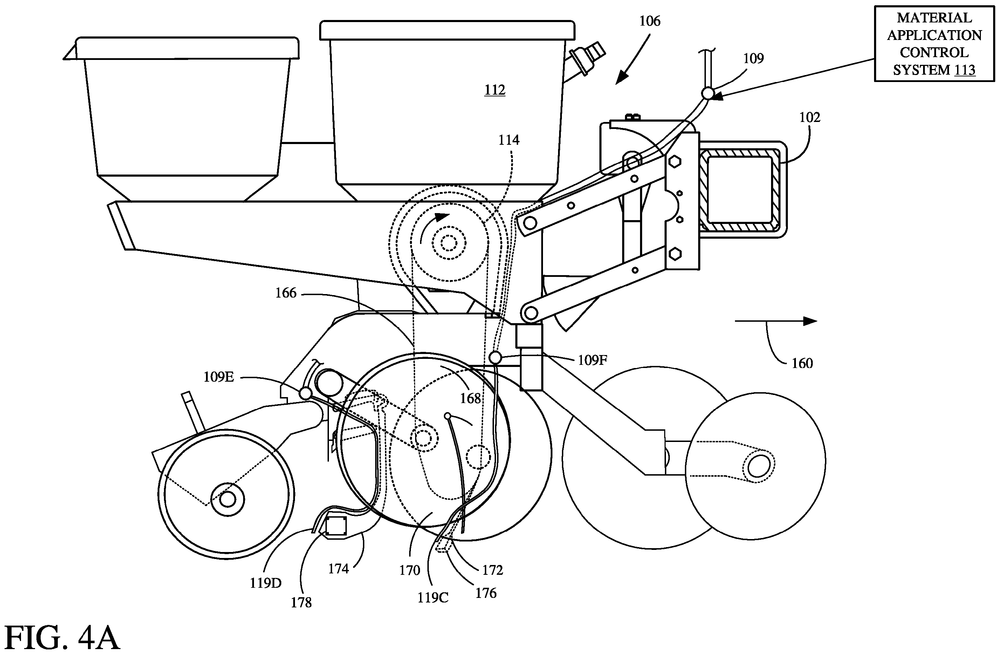

FIG. 4A is similar to FIG. 3 and similar items are similarly numbered. However, in FIG. 4A, row unit 106 is also provided with members 172 and/or 174. Members 172 and/or 174 can be spring biased into engagement with the soil, or rigidly attached to the frame of row unit 106. In one example, member 172 can be a furrow shaper, which contacts the soil in the area within or closely proximate the furrow, and immediately after the furrow is opened, but before the seed is placed therein. Member 172 can thus contact the side(s) of the furrow, the bottom of the furrow, an area adjacent the furrow, or other areas. It can be fitted with a sensor 176, e.g., seed sensor 176, as well.

In another example, member 172 can be positioned so that it moves through the furrow after the seed is placed in the furrow. In such an example, member 172 may act as a seed firmer, which firms the seed into its final seed location.

In either case, member 172 can include a seed sensor 176, which senses the presence of the seed. It may be an optical sensor, which optically senses the seed presence as member 172 moves adjacent to, ahead of, or over the seed. It may be a mechanical sensor that senses the seed presence, or it may be another type of sensor that senses the presence of the seed in the furrow. Sensor 176 illustratively provides a signal to material application control system 113 indicating the presence of the sensed seed.

In such an example, it may be that actuator 109 is placed at the location of actuator 109E, shown in FIG. 4A, and the outlet end of the application assembly is shown at 119C. In the example shown in FIG. 4A, outlet end 119C is shown closely behind member 172 relative to the direction indicated by arrow 160. It can be disposed on the opposite side of member 172 as well (such as forward of member 172 in the direction indicated by arrow 160). In such an example, the seed sensor senses the seed at a location that corresponds to its final seed location, or that is very closely proximate its final seed location. This may increase the accuracy with which seed sensor 176 senses the final seed location.

Also, in the example shown in FIG. 4A, row unit 106 can have member 174 in addition to, or instead of, member 172. Member 174 can also be configured to engage the soil within, or closely proximate, the trench or furrow. It can have a seed sensor 178 that senses the presence of a seed (or a characteristic from which seed presence can be derived). It can be placed so that it closely follows the exit end 121 of the seed tube 120, or the exit end 170 of the assistive delivery system 166. Also, actuator 109 can be placed at the position illustrated at 109F.

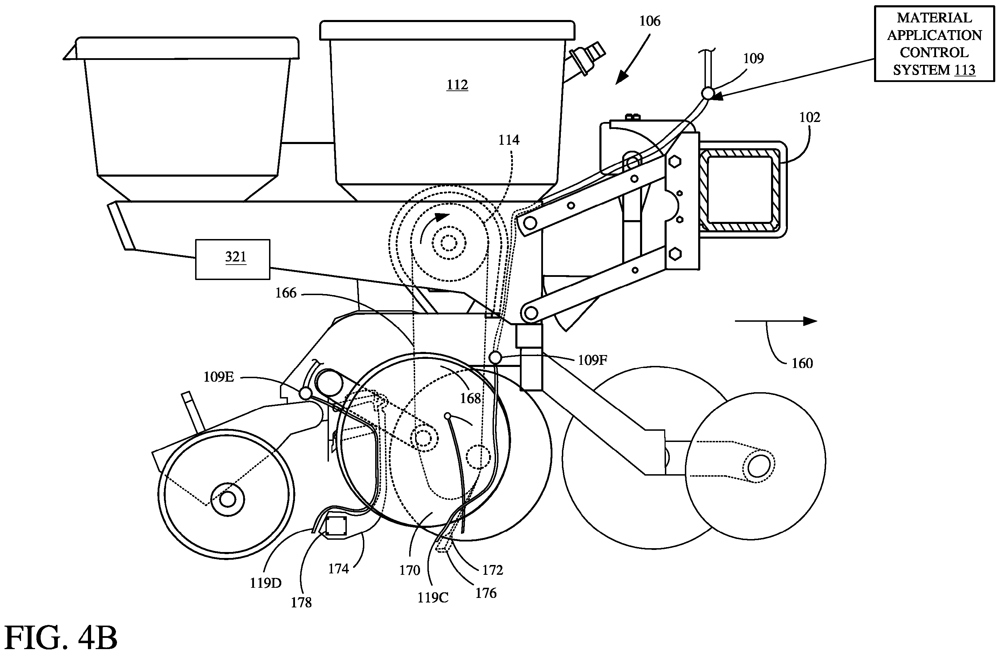

FIG. 4B is similar in some ways to FIG. 4A, and similar items are similarly numbered. FIG. 4B includes a moisture sensor 321. The moisture sensor 321 is configured to measure moisture levels of material such as ground material (e.g., soil). The moisture sensor 321 is configured to provide moisture data to the material application control system 113. The moisture sensor 321 may be communicatively coupled to the material application control system 113 such that the moisture sensor may continuously and/or intermittently provide data to the material application control system 321 throughout operation and/or after operation of the planting machine. The moisture data, which is based on measured moisture levels, may be processed and utilized by one or more processors such as the material application control system 113. As described above, the material application control system 113 may be configured to transmit the instructions to components of a planting machine (e.g. row unit 106), which may adjust operation of the planting machine. The material application control system may adjust a dispersion rate of water or fertilizer applied to a ground surface by sending instructions based at least in part on soil moisture data received from the moisture sensor 321. For example, the material application control system 113 may provide control data to the pumps 109 as described above by transmitting a signal thereto. The signal may be a signal indicating a desired amount of water or fertilizer to be disbursed with the commodity, based at least in part on a determined amount of moisture in the ground material.

The moisture sensor 321 may be a contact-based moisture sensor 321 or a non-contact-based moisture sensor. The contact-based moisture sensor 321 may be an insertable sensor. In the example shown in FIG 4B, the moisture sensor 321 is a non-contact moisture sensor. The moisture sensor 321 transmits radio waves to determine moisture levels. For example, the moisture sensor 321 may be a moisture sensor 321. In some examples, the moisture sensor 321 may be a contact-based moisture sensor.
The moisture sensor 321 may be coupled to an agricultural machine (e.g., row unit 106). In some examples, the moisture sensor 321 may be disposed about a surface of the agricultural machine such that a contact-based moisture sensor 321 may be lowered into a ground surface during a seeding operation. In some examples, the moisture sensor 321 may be positioned on the agricultural machine such that a non-contact-based moisture sensor may transmit signals (e.g., radio signals, electrical signals, etc.) into a ground surface to measure moisture levels in the ground surface during a seeding operation. For example, in FIG. 4B, the moisture sensor 321 is coupled to the sprayer and positioned to measure moisture levels in a ground surface during a seeding operation. However, in other examples, the moisture sensor may be coupled to other portions of a planting machine substantially adjacent a ground surface.

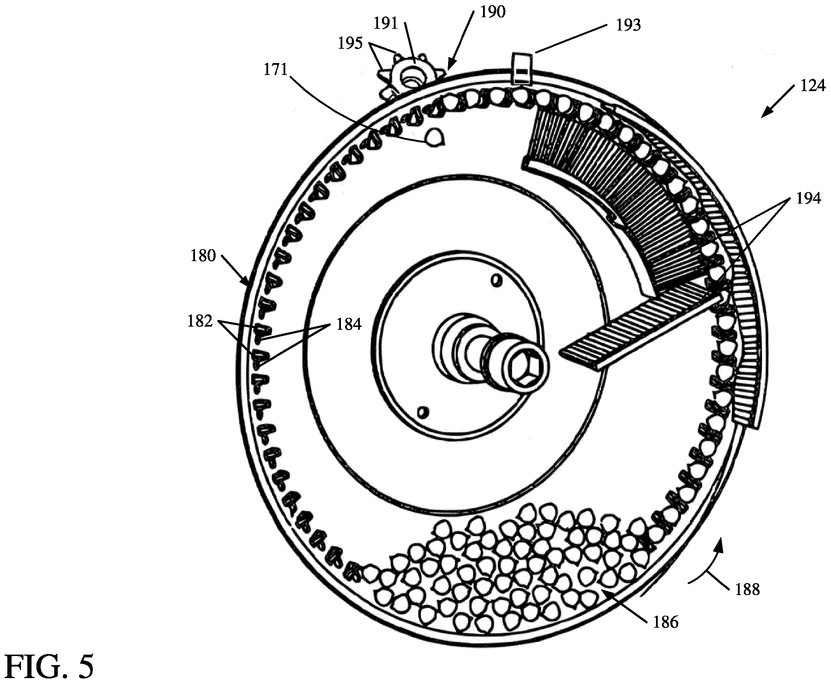

FIG. 5 shows one example of a rotatable mechanism that can be used as part of the seed metering system (or seed meter) 124. The rotatable mechanism includes a rotatable disc, or concave element, 180. Rotatable element 180 has a cover (not shown) and is rotatably mounted relative to the frame of the row unit 106. Rotatable element 180 is driven by a motor (not shown) and has a plurality of projections or tabs 182 that are closely proximate corresponding apertures 184. A seed pool 186 is disposed generally in a lower portion of an enclosure formed by rotating mechanism 180 and its corresponding cover. Rotatable element 180 is rotatably driven by its motor (such as an electric motor, a pneumatic motor, a hydraulic motor, etc.) for rotation generally in the direction indicated by arrow 188, about a hub. A pressure differential is introduced into the interior of the metering mechanism so that the pressure differential influences seeds from seed pool 186 to be drawn to apertures 184. For instance, a vacuum can be applied to draw the seeds from seed pool 186 so that they come to rest in apertures 184, where the vacuum holds them in place. Alternatively, a positive pressure can be introduced into the interior of the metering mechanism to create a pressure differential across apertures 184 to perform the same function.

Once a seed comes to rest in (or proximate) an aperture 184, the vacuum or positive pressure differential acts to hold the seed within the aperture 184 such that the seed is carried upwardly generally in the direction indicated by arrow 188, from seed pool 186, to a seed discharge area 190. It may happen that multiple seeds are residing in an individual seed cell. In that case, a set of brushes or other members 194 that are located closely adjacent the rotating seed cells tend to remove the multiple seeds so that only a single seed is carried by each individual cell. Additionally, a seed sensor 193 can also illustratively be mounted adjacent to rotating element 180. It generates a signal indicative of seed presence and this may be used by system 113, as will be discussed in greater detail below.

Once the seeds reach the seed discharge area 190, the vacuum or other pressure differential is illustratively removed, and a positive seed removal wheel or knock-out wheel 191, can act to remove the seed from the seed cell. Wheel 191 illustratively has a set of projections 195 that protrude at least partially into apertures 184 to actively dislodge the seed from those apertures. When the seed is dislodged (such as seed 171), it is illustratively moved by the seed tube 120, seed delivery system 166 (some examples of which are shown above in FIGS. 2-4 and below in FIGS. 6 and 7) to the furrow 162 in the ground.

FIG. 6 shows an example of a seed metering system and a seed delivery system, in which the rotating element 180 is positioned so that its seed discharge area 190 is above, and closely proximate, seed delivery system 166. In the example shown in FIG. 6, seed delivery system 166 includes a transport mechanism such as a belt 200 with a brush that is formed of distally extending bristles 202 attached to belt 200 that act as a receiver for the seeds. Belt 200 is mounted about pulleys 204 and 206. One of pulleys 204 and 206 is illustratively a drive pulley while the other is illustratively an idler pulley. The drive pulley is illustratively rotatably driven by a conveyance motor, which can be an electric motor, a pneumatic motor, a hydraulic motor, etc. Belt 200 is driven generally in the direction indicated by arrow 208

Therefore, when seeds are moved by rotating element 180 to the seed discharge area 190, where they are discharged from the seed cells in rotating element 180, they are illustratively positioned within the bristles 202 by the projections 182 that push the seed into the bristles 202. Seed delivery system 166 illustratively includes walls that form an enclosure around the bristles 202, so that, as the bristles 202 move in the direction indicated by arrow 208, the seeds are carried along with them from the seed discharge area 190 of the metering mechanism, to a discharge area 210 either at ground level, or below ground level within a trench or furrow 162 that is generated by the furrow opener 114 on the row unit 106.

Additionally, a seed sensor 203 is also illustratively coupled to seed delivery system 166. As the seeds are moved within bristles 202, sensor 203 can detect the presence or absence of a seed. It should also be noted that while the present description will proceed as having sensors 122, 193 and/or 203, it is expressly contemplated that, in another example, only one sensor is used. Or additional sensors can also be used. Similarly, the seed sensor 203 shown in FIG. 6 can be disposed at a different location, such as that shown at 203A. Having the seed sensor closer to where the seed is ejected from the system can reduce error in identifying the final seed location. Again, there can be multiple seed sensors, different kinds of seed sensors, and they can be located at many different locations.

FIG. 7 is similar to FIG. 6, except that seed delivery system 166 does not include a belt with distally extending bristles. Instead, it includes a flighted belt (transport mechanism) in which a set of paddles 214 form individual chambers (or receivers), into which the seeds are dropped, from the seed discharge area 190 of the metering mechanism. The flighted belt moves the seeds from the seed discharge area 190 to the exit end 210 of the flighted belt, within the trench or furrow 162.

There are a wide variety of other types of delivery systems as well, that include a transport mechanism and a receiver that receives a seed. For instance, they include dual belt delivery systems in which opposing belts receive, hold, and move seeds to the furrow, a rotatable wheel that has sprockets, which catch seeds from the metering system and move them to the furrow, multiple transport wheels that operate to transport the seed to the furrow, and an auger, among others. The present description will proceed with respect to an endless member (such as a brush belt, a flighted belt) and/or a seed tube, but many other delivery systems are contemplated herein as well.

Before continuing with the description of applying material relative to seed location, a brief description of some examples of seed sensors 122, 193 and 203 will first be provided. Sensors 122, 193 and 203 are illustratively coupled to seed metering system 124 and seed delivery system 120, 166. Sensors 122, 193 and 203 sense an operating characteristic of seed metering system 124 and seed delivery systems 120, 166. In one example, sensors 122, 193 and 203 are seed sensors that are each mounted at a sensor location to sense a seed within seed tube 120, seed metering system 124, and delivery system 166, respectively, as the seed passes the respective sensor location. In one example, sensors 122, 193, and 203 are optical or reflective sensors and thus include a transmitter component and a receiver component. The transmitter component emits electromagnetic radiation into seed tube 120, seed metering system 180, and/or delivery system 166. In the case of a reflective sensor, the receiver component then detects the reflected radiation and generates a signal indicative of the presence or absence of a seed adjacent to sensor 122, 193, and 203 based on the reflected radiation. With other sensors, radiation such as light, is transmitted through the seed tube 120, seed metering system 124, or the delivery system 166. When the light beam is interrupted by a seed, the sensor signal varies, to indicate a seed. Thus, each sensor 122, 193, and 203 generates a seed sensor signal that pulses or otherwise varies, and the pulses or variations are indicative of the presence of a seed passing the sensor location proximate the sensor.

For example, in regards to sensor 203, bristles 202 pass sensor 203 and are colored to absorb a majority of the radiation emitted from the transmitter. As a result, absent a seed, reflected radiation received by the receiver is relatively low. Alternatively, when a seed passes the sensor location where sensor 203 is mounted, more of the emitted light is reflected off the seed and back to the receiver, indicating the presence of a seed. The differences in the reflected radiation allow for a determination to be made as to whether a seed is, in fact, present. Additionally, in other examples, sensors 122, 193, and 203 can include a camera and image processing logic that allow visual detection as to whether a seed is present within seed metering system 124, seed tube 120, and/or seed delivery system 166, at the sensor location proximate the sensor. They can include a wide variety of other sensors (such as RADAR or LIDAR sensors) as well.

For instance, where a seed sensor is placed on a seed firmer, it may be mechanical or other type of sensor that senses contact with the seed as the sensor passes over the seed. Also, while the speed of the seed in the delivery system (or as it is ejected) can be identified by using a sensor that detects the speed of the delivery system, in some examples, the speed and/or other characteristics of movement of the seed can be identified using seed sensors. For instance, one or more seed sensors can be located to sense the speed of movement of the seed, its trajectory or path, its instantaneous acceleration, its presence, etc. This can be helpful in scenarios in which the seed delivery system changes speed.

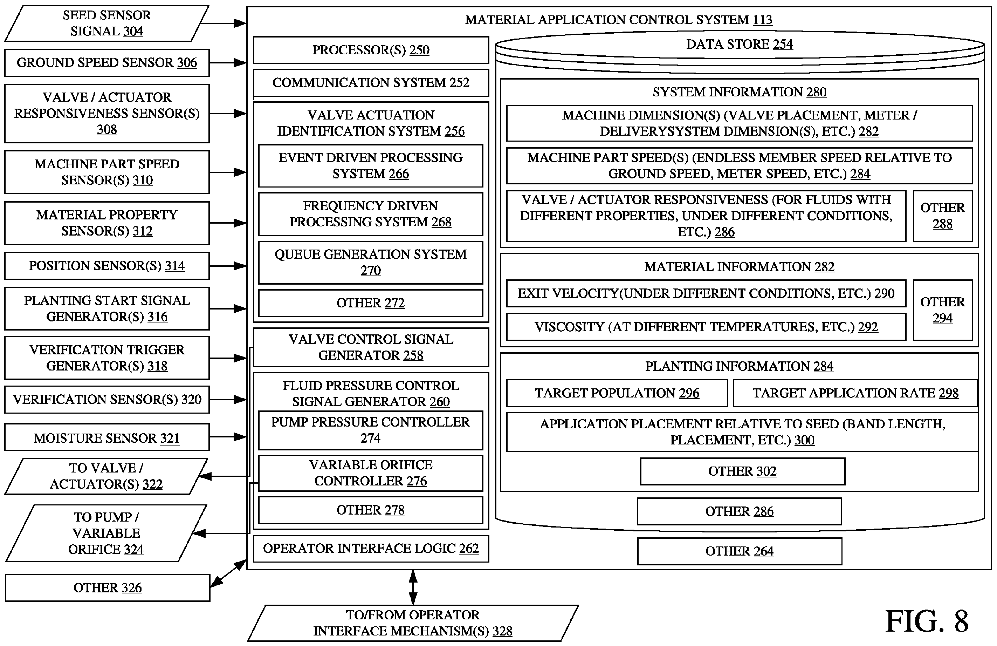

FIG. 8 is a block diagram showing one example of material application control system 113 in more detail. In the example, it is assumed that actuators 109 are valves and that the material to be applied is pumped to actuators 109 by pump 115. Of course, this is just one example and actuators 109 could be pumps or other actuators as well. In the example shown in FIG. 8, system 113 illustratively includes one or more processors 250, communication system 252, data store 254, valve actuation identification system 256, valve control signal generator 258, fluid pressure control signal generator 260, operator interface logic 262, and it can include a wide variety of other items 264. FIG. 8 also shows that valve actuation identification system 256 can include event driven processing system 266 and/or frequency driven processing system 268. It illustratively includes queue generation system 270, and it can include a wide variety of other items 272. Fluid pressure control signal generator 260 illustratively includes pump pressure controller 274 and/or variable orifice controller 276. It can include other items 278 as well.

Data store 254 can include a wide variety of different types of information. The information can be pre-configured or pre-programmed into data store 254, or it can be sensed by sensors and stored in data store 254 intermittently (such as periodically, or it can be regularly updated based on those sensor inputs). By way of example, data store 254 illustratively includes system information 280, material information 282, planting information 284, and it can include a wide variety of other information 286. System information 280 illustratively includes information about the planter 100, the delivery system 120, 166, and/or other items that are used to plant seed. It includes information that can be used to identify when to apply material relative to the seed location of a seed in furrow 162. Therefore, it can include information that allows valve actuation identification system 256 to identify a timing of when the valves 109 should be opened to apply the material, relative to the seed location.

As examples, system information 280 can include machine dimensions 282. These dimensions can include dimensions that indicate where the valve is placed relative to the outlet opening of the seed delivery system. It can include dimensional information indicating where the valve is placed relative to the seed sensor. It can include information such as the size, e.g., one or more dimensions, of the seed delivery system 120, 166, so that the distance between the seed sensor and the furrow 162 can be identified. It can include a wide variety of other machine dimension information 282 as well.

System information 280 can also include machine part speed information 284. This information can include the speed of an endless member used to deliver seed (such as the brush belt 200 delivery system or the flighted belt 214 delivery system). Where the speed of those mechanisms changes with the ground speed of the planter, information 284 can identify the different belt speeds relative to this sensed ground speed or provide a correlation so the belt speed can be calculated given the ground speed. It can include other machine part speed information as well such as the speed of metering system 124, etc.

System information 280 also illustratively includes valve/actuator responsiveness information 286. Information 286 can indicate the responsiveness of the actuator 109 that is being used to apply the material. In one example, the actuator 109 may be a solenoid valve so that there is a latency between when a valve open signal is applied and when the solenoid actually opens the valve. The same is true for closing the valve. That is, there may be a latency between when the valve close signal is applied and when the valve actually closes. In addition, the actuator responsiveness may change based upon the particular properties of the material that is flowing through the valve. It may change based upon the type of nozzle that is being used, and it may change under different ambient conditions (e.g., it may take longer to cycle when the weather is cold than when the weather is warm, etc.). The valve/actuator responsiveness information 286 can indicate valve responsiveness given these and other types of information. System information 280 can include a wide variety of other information 288, as well.

Material information 282 illustratively identifies properties of the material that is being applied by the system. For instance, material information 282 may include an exit velocity information 290 that identifies a velocity at which the material exits the valve or actuator or nozzle that is being used to apply it. Again, the exit velocity of the material may change based on the material, under different conditions, and the exit velocity information 290 may indicate this as well.

Where the material is a liquid material, then material information 282 may also include viscosity information 292, which identifies the viscosity or other liquid properties of the material. The viscosity may change at different temperatures or under other circumstances, and viscosity information 292 will illustratively indicate this. The material information 282 can include a wide variety of other information 294 indicative of other properties of the material being applied.

Planting information 284 can include a wide variety of different types of information indicative of the planting operation. For example, it can include target population information 296 that identifies a target seed population. It can include target application rate information 298 that indicates a target application rate for the material being applied. It can include application placement relative to seed information 300 that indicates placement properties of the application, or application pattern, for the material. For instance, where the material is liquid material and is being applied in a band of liquid, it may indicate the length of each application band to be applied by the valve or actuator. It may indicate a placement of that band relative to the seed location. For instance, where the band is to be four inches long, the placement information may indicate a relative placement of the center of the band (along its longitudinal length) relative to seed location. In this way, where the material is to be applied at the seed location, then the center of the band will illustratively correspond to the seed location. However, where the material is to be applied at a location other than the seed location, then the center of the band will illustratively be offset from the seed location by a desired amount. Similarly, the application rate may vary within an application band. For instance, the material may be applied more heavily near the center of the band than at either end of the band or vice versa. This type of information can be included in information 300. The planting information 284 can include a wide variety of other information 302, indicative of the planting operation, as well.

FIG. 8 also shows that material application control system 113 illustratively receives one or more seed sensor signals 304 that may be generated from one or more of seed sensors 122, 193 or 203, or another seed sensor located at a different location. Seed sensor signal 304 may illustratively indicate when the particular sensor senses the presence of a seed.

FIG. 8 also shows that, in one example, system 113 includes an input from ground speed sensor 306, which senses the ground speed of row unit 106. The sensor may be located on the towing vehicle or elsewhere, and illustratively provides a sensor signal indicative of ground speed.

FIG. 8 also shows that in some examples, the material application control system 113 includes an input from the moisture sensor 321, which senses a moisture level of a ground material as described above. The moisture sensor 321 may be disposed about the sprayer as described above and shown in FIG. 4B, or elsewhere on a planting machine. The moisture sensor 321 illustratively provides a sensor signal indicative of moisture levels of ground material. However, it is contemplated that the moisture sensor 321 may provide one or more sensor signals indicative of other moisture levels.

As discussed above, some of the information stored in data store 254 may be pre-configured or pre-defined. In another example, it may be sensed by various sensors. Therefore, in one example, system 113 receives the valve/actuator responsiveness information 286 from a valve/actuator responsiveness sensor 308. Sensor 308 may illustratively sense the movement of a solenoid, or other actuator, to sense how responsive the actuator or valve is to the control signals that are applied to it. Thus, it may provide a signal indicative of the latency between applying a valve open signal (or pump on signal) and when the valve actually opens (or the pump turns on), and indicative of latency between applying a valve close signal (or pump off signal) and when the valve actually close (or the pump turns off), among other things.

The machine part speeds information 284 may also be sensed by machine part speed sensors 310. Those sensors may illustratively sense the speed at which the seed delivery system 166 is moving, the speed at which the seed metering system 124 is moving, or the speed of a wide variety of other machine parts that are needed to generate the actuator signals used to apply material, as desired.

Similarly, the material information 282 can be sensed as well, by material property sensors 312. Those sensors may sense such things as material temperature, material viscosity, among other things.

System 113 can also receive an input from a position sensor 314. Position sensor 314 may include a GPS system, a LORAN system, a cellular triangulation system, or another type of position system that provides a signal indicative of the position of the sensor 314 in a global or local coordinate system. Such a sensor can also be used to determine ground speed, among other things.

System 113 also illustratively receives an input from a planting start signal generator 316. Generator 316 can cause any of a wide variety of different signals from any of a wide variety of different sensors, actuators or control inputs, indicating that the planting operation has begun. For instance, it may be that the operator 92 of the towing vehicle 94 depresses a button or provides another input through a user interface mechanism 96 to start the planting operation. This may be sensed by signal generator 316 and used to generate a signal indicating that the planting operation has begun. In another example, generator 316 may include a sensor indicating that either metering system 124 or seed delivery system 166 has begun moving. In another example, generator 316 may include a sensor indicating that a seed has passed the sensors 122, 193 and/or 203. It may generate a signal based on this, which is indicative of the planting operation beginning.

System 113 can also receive an input from verification trigger generator 318. Generator 318 provides an input, under certain circumstances, which are described in greater detail elsewhere herein, indicating when system 113 is to perform a verification operation, especially where frequency driven processing system 268 is used.

When a verification operation is to be performed, signals may be received by system 113 from a variety of different verification sensors 320. Examples of these sensors are described elsewhere herein.

Valve control signal generator 258 illustratively generates control signals 322 that are sent and/or applied to the valves or actuators 109 in order to apply the material. This is also described in greater detail herein.

In some examples, the material being applied is a fluid that is provided as fluid under pressure by pump 115. In that case, the pump displacement may be controlled to control the pressure. Similarly, the valves or nozzles may be provided with a variable orifice. In that case, the variable orifice may be controlled as well. Therefore, fluid pressure control signal generator 260 illustratively generates outputs 324 that illustratively control the pump and/or variable orifice, in those scenarios. In a scenario in which actuators 109 are pumps, outputs 324 can control those pumps as well.

System 113 can include a wide variety of other inputs and it can generate a wide variety of other outputs as well. This is indicated by block 326.

FIG. 8 also shows that, in one example, operator interface logic 262 may generate signals that are output to operator interface mechanisms 96, and it can receive information from those mechanisms as well. This is indicated by block 328.

Before describing the overall operation of material application control system 113, a brief description of some of the items in system 113 will first be provided. Communication system 252 can be any of a wide variety of different types of communication systems that allow material application control system 113 to communicate with a control system on towing vehicle 94 and/or operator interface mechanisms 96. It can also allow items on system 113 to communicate with one another, and to communicate with one or more different remote computing systems. Therefore, for instance, communication system 252 can include a controller area network - CAN - communication system, a local area network, a wide area network, a near field communication system, a cellular communication, or any of a wide variety of other networks or combinations of networks and communication systems.

Valve actuation identification system 256 illustratively receives some of the inputs discussed above and identifies when the valves 109 are to be actuated in order to apply material at a desired location relative to the location of the seeds being placed in furrow 162. In one example, it includes event driven processing system 266, which determines when the valves are to be actuated based on an event, such as based upon receiving an indication from seed sensors signal 304 that a seed has been sensed or receiving an indication from the moisture sensor 321 that a desired amount of material is to be disbursed. For instance, referring to FIG. 3, assume that seed sensor 122 detects a seed in delivery system 166. Event driven processing system 266 calculates a time that it will take that seed to travel to the outlet end 170 of delivery system 166, based upon the speed of delivery system 166, as indicated by machine part speed sensors 310. It then calculates a time delay, during which the seed will move from the outlet end 170 of the seed delivery system 166 to its final resting place is furrow 162. Then, based upon the location of the valve 109 on row unit 106, the valve/actuator responsiveness, the exit velocity or viscosity of the material being applied, the ground speed of row unit 106, and based upon the desired placement of the material relative to the seed location (as indicated by the corresponding information in data store 254 or based on the inputs from the sensors discussed above such as the moisture sensor 321), event driven processing system 266 calculates when the valves 109 should be actuated in order to apply the material at the desired place, relative to the seed location.

In another example, queue generation system 270 generates a set of valve actuation timing signals, indicating when valves 109 should be actuated, for a future sequence of actuations. For instance, queue generation system 270 may generate a queue of timing signals that are generated either by event driven processing system 266 or frequency driven processing system 268, and provide that plurality of queued timing signals to valve control signal generator 258. Valve control signal generator 258 can receive that set of signals and generate valve actuator control signals based upon the queued sequence of timing signals. In this way, the network bandwidth for communication between valve actuation identification system 256 and valve control signal generator 258 need not be as high. By communicating a plurality of valve actuation timing signals as a queued sequence of signals, the frequency with which those signals need to be sent can be greatly reduced over an implementation in which each valve actuation timing signal is sent, individually and separately, for each valve actuation.

Valve control signal generator 258 can generate the valve control signals in a wide variety of different ways. It can generate those signals and apply them through a hardware wiring harness, through wireless communication, or in other ways.

In some examples, the fluid pressure of the material to be applied is to be controlled. For instance, by increasing the fluid pressure, this may increase the exit velocity of the material as it is applied by the valve or nozzle being controlled. Similarly, where the valve or nozzle is not directed vertically, but has a horizontal component to its orientation, increasing the fluid pressure may change the trajectory of the fluid after it exits the valve or nozzle. This will change the location on the ground where the material is applied.

In the same way, where the valve or nozzle is provided with a variable orifice, varying the orifice may change the trajectory or exit velocity of the material as well. Thus, pump pressure controller 274 can control the pump pressure to obtain a desired exit velocity and/or trajectory of the material being applied. Variable orifice controller 276 can variably control the orifice to also achieve a desired exit velocity and/or trajectory of the applied material. In some examples, variable orifice controller 276 and pump pressure controller 274 can work in concert to control the exit velocity and/or trajectory of the material being applied.

Operator interface logic 262 can generate information that is provided to operator interface mechanisms 96 so that operator 92 can interact with that information. Similarly, operator interface logic 262 can receive information indicative of operator inputs from operator 92 through operator interface mechanisms 96. It can communicate that information to the various items or components on/of material application control system 113.

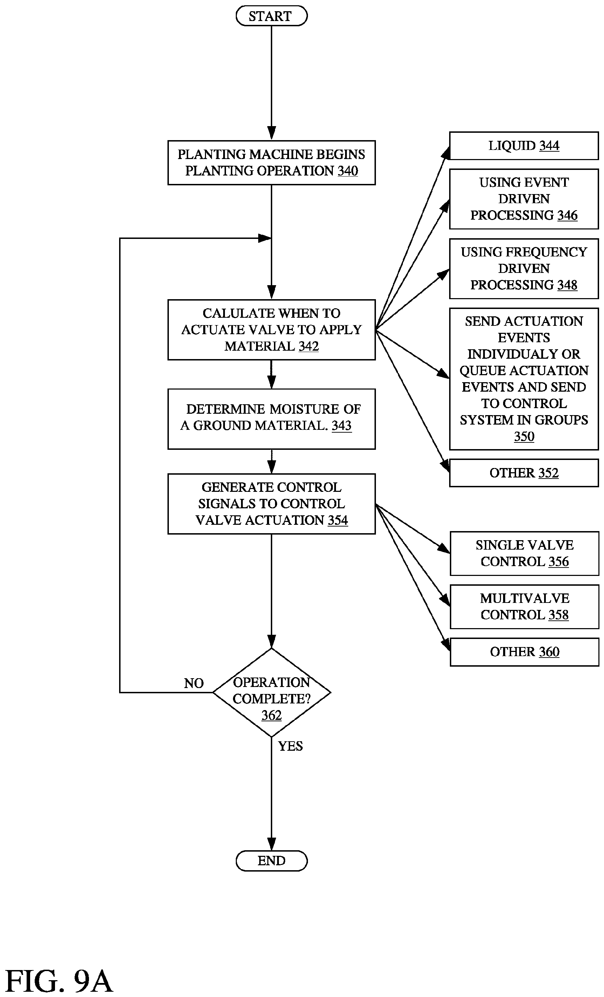

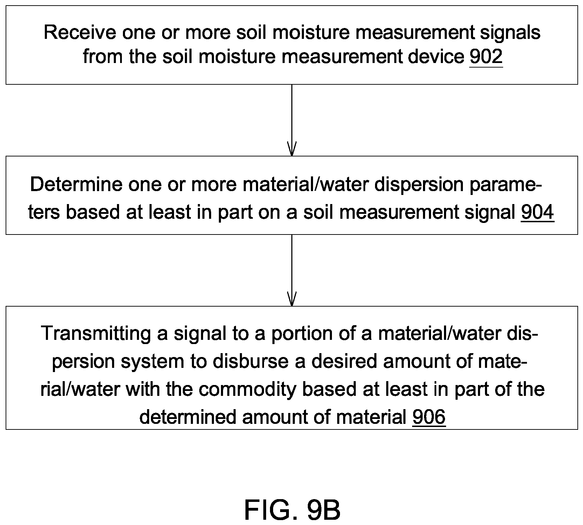

FIG. 9A is a flow diagram illustrating one example of the overall operation of material application control system 113. It is first assumed that the planting machine (e.g., row unit 106) begins the planting operation. This is indicated by block 340 in the flow diagram of FIG. 9. Based upon the various inputs, valve actuation identification system 256 calculates when to actuate the valves to apply the material relative to the seed location in the furrow 162. This is indicated by block 342. In one example, the material can be liquid. This is indicated by block 344. In one example, system 256 can determine when to actuate the valves using event driven processing system 266. This is indicated by block 346 in the flow diagram of FIG. 9. In another example, system 256 can determine when the valves are to be actuated using frequency driven processing system 268. This is indicated by block 348. In one example, frequency driven processing system 268 can be used in conjunction with event driven processing system 266. Further, the timing indicators, indicating when the valves 109 are to be actuated, may be sent individually or they may be grouped together by queue generation system 278 and sent, as groups, to valve control signal generator 258. Sending them individually or queuing the actuation events and sending them to valve control signal generator 258, in groups, is indicated by block 350 in the flow diagram of FIG. 9A. Valve actuation identification system 256 can identify when the valves are to be actuated and output an indication of that to valve control signal generator 258, in other ways as well. This is indicated by block 352 in the flow diagram of FIG. 9A. At block 343, the material application control system 113 determines a moisture of a ground material based at least in part on one or more sensor signals from the moisture sensor 321.

Valve control signal generator 258 generates control signals to control valve actuation, based upon the output from valve actuation identification system 256. The control signals control valves 109 so that the material being applied is applied at the desired location in the furrow, e.g., relative to the seed location. Generating control signals to control valve actuation is indicated by block 354 in the flow diagram of FIG. 9A.

In one example, there is a single valve that controls a single nozzle, per row being planted. Controlling a single valve is indicated by block 356. In another example, it may be that the nozzles or valves do not have a high enough bandwidth, e.g., max operating frequency, in order to apply the material at the desired frequency. In that case, there may be multiple valves per row being planted that can be controlled in order to achieve a higher application rate. These same types of configurations can be used when actuators 109 are pumps instead of valves.

For example, the seed population (e.g., seeds/acre) and row spacing are used to determine the seed-to-seed spacing in the furrow 162. This spacing, and the travel speed of planter 100, can be used to identify how quickly a valve is to respond in order to administer per-seed application (e.g., on each seed or between adjacent seeds/grains). A target seed rate of, for instance, 36,000 seeds/acre, with the planter traveling at 10 mph and with a thirty-inch row spacing, means that a nozzle on the planter will pass thirty seeds per second, or one seed every thirty three milliseconds. Some current fertilizer valves operate at about 10-15 Hz. Additionally, the opening time and closing time for some current valves can be approximately 7-8 milliseconds. This is often not fast enough to place fertilizer (or other material) on a per seed basis. Thus, the present system can have two or more valves (e.g., solenoids or other valves) per row operating out of phase (e.g., evenly out of phase) with one another to increase the overall frequency with which material can be applied in a row. While one valve is closing, another can be opening. Each valve can have its own nozzle or multiple valves can share a nozzle or multiple valves can provide material to multiple nozzles. These multiple valves per row can be placed proximate one another in the valve locations identified above in FIGS. 2-4 or elsewhere. They can be controlled using control signals timed as described herein for a single valve, except that the valve control signals can be spread across the multiple valves to obtain the desired material application rate, timing and placement.

Further, it may be desirable to apply multiple different materials per row. In that case, there may be multiple different valves, per row, each dispensing a different material. Thus, valve actuation identification system 256 can provide an indication as to when to actuate each of the valves to apply the corresponding material, so that it is applied at the desired location relative to the seed, and valve control signal generator 258 generates control signals for the different valves, based upon the output from system 256. For example, one valve or valve set may apply a first commodity directly to the seed while another valve or valve set may apply a second commodity, e.g., a hot commodity, between seeds. Controlling multiple valves per row is indicated by block 358. Valve control signal generator 258 can generate control signals to control the valves in a wide variety of other ways as well, and this is indicated by block 360.

This continues until the planting operation is complete. This is indicated by block 362 in the flow diagram of FIG. 9A.

FIG. 9B shows a flow chart illustrating example steps for at least a portion of operation of the material application control system 113 to determine moisture of a ground material 343. In some examples, the material application control system 113 is configured to cause the seed dispersion system to disburse moisture and/or material such as fertilizer onto or adjacent a commodity based on the moisture of the ground material. The material application control system 113 may be further configured to receive one or more soil moisture measurement signals from the soil moisture measurement device 115.

For example, material application control system 113 system may distribute moisture and/or material such as fertilizer based at least in part on one or more measurements taken by the soil moisture measurement device. As such, the material application control system 113 is configured to promote seed growth in various and changing soil moisture conditions.

The material application control system 113 may adjust dispersion to maximize growth in a plurality of soil compositions during a substantially continuous seeding operation. In some examples, the material application control system 113 is further configured to utilize soil composition data obtained during a seeding operation. The soil composition data (e.g., soil type data) may be received from various data sources, such as sensors or database resources. In some examples, the material application control system 113 may further base material dispersion on additional data such as previously recorded soil composition data and/or soil composition data. In some examples, the material application control system 113 may be further configured to utilize precipitation data, such as past or predicted weather data related to precipitation at a ground location.

The material application control system 113, may be configured to receive data related to the soil moisture to the material application control system 113. In some examples, the material application control system 113 may deliver data based on instructions from the material application control system 113 to provide measurements according to desired data measurement parameters. For example, the material application control system 113 may deliver data related to soil moisture, continuously or over certain time intervals, distance intervals, or based on other operational parameters.

At 902, the material application control system 113 determines one or more dispersion parameters such as water dispersion parameters or fertilizer dispersion parameters. The one or more dispersion parameters may be based on one or more qualities related to promoting growth and germination of commodity. In some examples water dispersion parameters may include, water amount, water location (e.g., next to commodity, upon commodity, over commodity, etc.), water pattern (e.g., spray, stream, etc.), and dispersion force. In some examples, the water dispersion parameters include a moisture differential, which may be a difference between a desired moisture of a ground material and measured moisture of the ground material. In the example process shown in FIG. 9, the one or more dispersion parameters are based at least in part on a soil moisture measurement signal from the moisture measurement device 115.

At 904, the material application control system 113 determines one or more material dispersion parameters. In some examples, the material dispersion parameters may be water dispersion parameters or fertilizer dispersion parameters. In some examples where the material application control system determines fertilizer dispersion parameters, the dispersion parameters may be fertilizer dispersion parameters. The fertilizer dispersion parameters may be parameters based at least in part on moisture measurement and related to fertilizer composition. For example, the fertilizer dispersion parameters may include one or more of a time between dispersion, a duration of dispersion, and an amount of dispersion, which may be optimized for a chemical composition of a fertilizer being disbursed.

In some examples, the fertilizer dispersion parameters may also include synchronization parameters with respect to commodity being distributed. For example, the material application control system 113 may utilize synchronization parameters to determine whether to disburse fertilizer directly onto a commodity as it is being planted or to disperse the commodity adjacent the commodity as it is being planted as described above. As such, in some examples, the material application control system 113 may utilize the dispersion parameters to determine to disburse commodity and fertilizer in alternating time intervals, in simultaneous time intervals, in direct contact or adjacent each other in a furrow during a planting operation.

In examples where the material application control system 113 determines water dispersion, the dispersion parameters may be water dispersion parameters. The water dispersion parameters may be parameters based at least in part on moisture measurement related to water compensation data which may be data provided to raise the moisture about a commodity to a desired level. For example, the water dispersion parameters may include one or more of a time between dispersion, a duration of dispersion, and an amount of dispersion which may be optimized based at least in part on desired ground saturation. In some examples, the water dispersion parameters may also include synchronization parameters with respect to commodity being distributed. For example, the material application control system may utilize synchronization parameters to determine whether to disburse water directly onto a commodity as it is being planted or to disburse the water next to the commodity as it is being planted. As such, in some examples, the material application control system 113 may utilize the dispersion parameters to determine to disburse commodity and a water in alternating time intervals, in simultaneous time intervals, directly in direct contact or adjacent each other in a furrow.

The dispersion parameters may be based at least in part on one or more of a plurality of contributing parameters. For example, the dispersion parameters may be based at least in part on soil identification data such as predetermined soil composition data. In some examples, soil composition data may include chemical composition of soil typically found at a particular location. The soil composition data may be based on previously recorded soil indicating chemical composition.

In some examples, one or more water dispersion parameters or fertilizer dispersion parameters may be based at least in part on a soil moisture measurement signal. However, in some examples, the one or more water dispersion parameters may be based on other factors such as ground speed and distance traveled as described above.

At 906 the material application control system 113 transmits a signal to the one or more of a water dispersion system or a fertilizer dispersion system. The signal may be used to disburse a desired amount of water or fertilizer based at least in part on the determined water or fertilizer dispersion parameter. As such, the material application control system can generate signals at least in part based on one of more of the dispersion parameters to control operation of a planting machine in real time.

In some examples, the material application control system 113 may utilize measured moisture data to determine operational parameters in future operations or an active operation. In some examples the material application control system may be used to train one or more machine learning systems to predict moisture levels in a ground material.

In some examples, the material application control system 113 may record measured moisture levels in a plurality of ground positions. The material application control system 113 may further associate and/or determine location of moisture as determined by one or more location systems such as GPS. In some examples, machine learning and or algorithm-based techniques maybe implemented to analyze the measured moisture levels. For example, machine learning and or algorithm-based techniques may be used to transform and/ associate disparate data forms from location and moisture date to provide one or more moisture maps. The one or more moisture maps may be used for subsequent seeding operations.

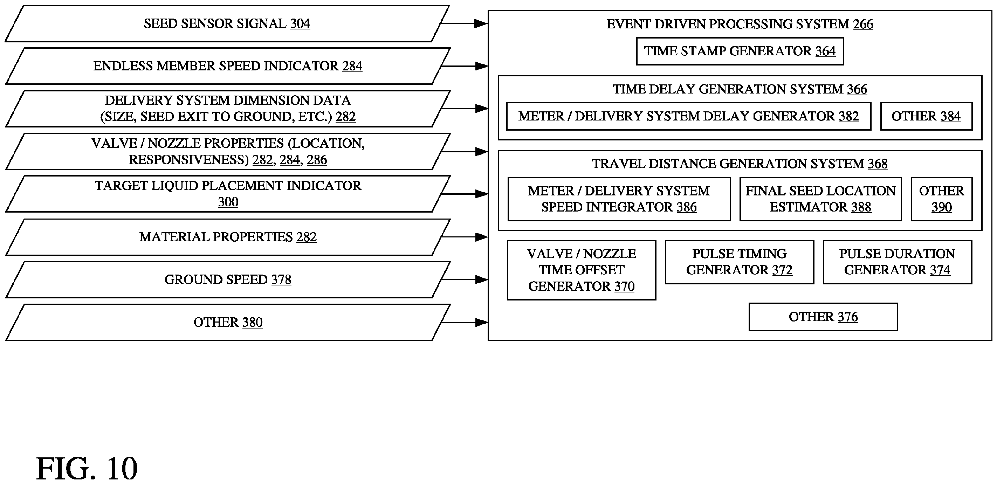

FIG. 10 is a block diagram showing one example of event driven processing system 266, in more detail. System 266 illustratively includes time stamp generator 364, time delay generation system 366, travel distance generation system 368, valve/nozzle time offset generator 370, pulse timing generator 372, pulse duration generator 374, and it can include a wide variety of other items 376. FIG. 10 also shows that, in one example, event driven processing system 266 receives the one or more seed sensor signals 304, and endless member speed indicator 284 that indicates the speed of the delivery system 166 and/or meter 124, delivery system and meter dimension data 282 that indicates such things as the meter and/or delivery system size, the distance that the seed will drop after it exits the delivery system to the furrow, etc. System 266 receives valve/nozzle properties (such as valve location and responsiveness). This corresponds to the information 282, 284, and 286 discussed above. It can receive target liquid placement indicator 300, that identifies where the material is to be applied, e.g., relative to the seed in the furrow. It can receive the material properties 282. It can also receive an input indicative of ground speed 378. This can come from ground speed sensor 306 (discussed with respect to FIG. 8) or elsewhere. It can receive a wide variety of other information 380 as well. As discussed above, the inputs to event driven processing system 266 can include information from data store 254 or from various different sensors, or from a combination of those things.

Time stamp generator 364 illustratively receives seed sensor signal 304 and generates a time stamp indicating when signal 304 indicates the presence of a seed. Time delay generation system 366 then generates a time delay indicative of the amount of time it will take the seed to travel from the particular seed sensor that sensed it, to an outlet opening of the seed delivery system 166 or seed tube 120. For instance, meter/delivery system delay generator 382 illustratively calculates the amount of time it will take the seed to travel from wherever it was sensed (meter 124, seed delivery system 120 or 166, or elsewhere) to the exit end of the seed delivery system, based upon the type of seed delivery system, the type of meter, etc. Where the seed is sensed in the seed meter, generator 382 obtains the speed of the seed meter and the speed of the delivery system 166, along with the size of both (e.g., the distance the seed must travel at the two different speeds) to identify a time when the seed will exit the seed delivery system. If the seed delivery system 166 is a seed tube 120, then the travel time through the seed tube will correspond, at least partially, to the acceleration of gravity, as the seed passes through the seed tube (other factors may impact the calculation, such as the forward travel speed of the row unit 106 and the coefficient of friction of both the seed and the inner surface(s) of the seed tube, among others). If the delivery system 166 is an assistive system, then the time will include the delay of that system in moving the seed from wherever it was sensed, to the outlet end of the delivery system. This may be based on the speed of an endless member, the velocity of a pneumatic assistance system, etc. If the seed sensor is located closer proximate the outlet end of the delivery system, then the time calculated by meter/delivery system delay generator 382 will be less, because the seed will be detected closer to the exit end of the delivery system. Depending on the response time of the valves 109, a seed sensor may be located as close as possible to the outlet end of the delivery system (or elsewhere, such as on a seed firmer) to minimize, if not eliminate, the time calculated by meter/delivery system delay generator 382. System 366 can include other items 384 as well.

In the example where time delay generation system 366 identifies the time delay, then valve/nozzle time offset generator 370 illustratively calculates a time offset corresponding to the responsiveness of the valve being controlled, under the current conditions. For instance, the responsiveness may vary based upon the particular valve or actuator, based on the properties of the material being applied, based upon the ambient conditions, based on the pump pressure, or based on other things. Valve/nozzle time offset generator 370 generates an output indicative of a time offset that corresponds to a latency in actuation of the valve.

Pulse timing generator 372 then generates a timing output indicative of a time when the valve should be actuated, based upon the time delay generated by meter/delivery system delay generator 382 and the offset generated by valve/nozzle time offset generator 370. In short, meter/delivery system delay generator 382 calculates the amount of time it will take for the seed to move from where it was sensed to its final seed location in furrow 162, and valve/nozzle time offset generator 370 will calculate how long it will take to begin applying the material, based upon the properties of the valve or actuator, once the valve actuation control signal is applied. Pulse timing generator 372 then generates a time when the actuator control signal should be applied to the valve/actuator, based upon the delay time generated by generator 382 and the offset generated by generator 370, and based upon the time stamp generated by time stamp generator 364.

Pulse duration generator 374 generates an output indicative of how long the valve should stay on, e.g., open. This can include determining the latency in the valve response between the time that it is commanded to close and when it actually closes. This may vary based upon the type of material being applied, based upon ambient conditions, etc. The two timing signals (the pulse time indicating when the actuator should be actuated, and generated by generator 372, and the pulse duration output by pulse duration generator 374) are provided to valve control signal generator 258. Valve control signal generator 258 generates valve control signals to actuate the valve at the time indicated by pulse timing generator 372, and to keep the valve actuated for a duration indicated by pulse duration generator 374. The rate at which the material is applied can also be varied. For example, the valve may be a proportional valve so more or less material can be applied.

In another example, where the seed metering system or delivery system changes speeds during planting, instead of using time delay generation system 366 (or in addition to it), event driven processing system 266 can include travel distance generation system 368. Instead of calculating the time that will be needed to move the seed from the location where it was sensed, e.g., in the meter or the seed delivery system, into the furrow, meter/delivery system speed integrator 386 can integrate acceleration (that arises from changes in velocity) to obtain the velocity of the system and it can then integrate the velocity to obtain a signal indicative of the location of the seed (or the distance it has traveled) to determine when the seed exits the seed delivery system, and final seed location estimator 388 determines where the seed will be in its final seed location in furrow 162.

As an example, meter/delivery system speed integrator 386 integrates the acceleration and speed of the seed meter (if the seed sensor is disposed on the seed meter), and/or the acceleration and speed of the seed delivery system 166 (if the seed sensor is disposed on the seed delivery system). This provides a distance indicator that indicates the distance traveled by the seed. This distance is compared to the dimensions of the meter and/or delivery system 166 to determine when the seed has traveled a sufficient distance that it has reached the exit end of the seed delivery system 166. When this occurs, integrator 386 provides an output indicating that the seed should now be exiting the seed delivery system 166.

Final seed location estimator 388 estimates when the seed has reached its final location based upon the distance it must travel from the exit end of the seed delivery system 166 to the bottom of the furrow 162. It may include factors that accommodate for possible rolling of the seed, or other movement of the seed after it reaches the ground. Final seed location estimator 388 then generates an output indicating that the seed has reached its final location.

Knowing that the seed is in its final location, valve/nozzle time offset generator 370 can generate an output indicating any time offset corresponding to the valve/actuator latency or responsiveness. Pulse timing generator 372 and pulse duration generator 374 generate the timing for the valve/actuator control signal and its duration, as described above.

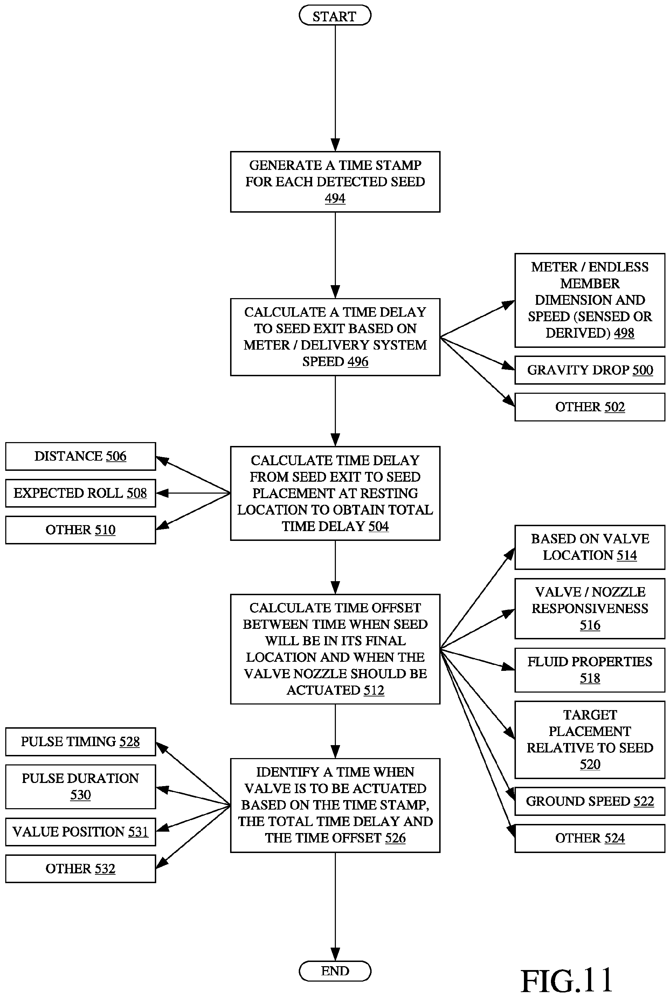

FIG. 11 shows that system 268 can illustratively receive a variety of inputs, some of which are the same as those discussed above, and are similarly numbered. System 268 also illustratively receives a planting start indicator 534 that can be generated from planting start signal generator 316. Some examples of this are discussed elsewhere herein. It can receive a verification trigger 536 generated from verification trigger generator 318. It can also receive verification sensor inputs 538 that are generated by verification sensors 320. It can receive a wide variety of other information 540 as well.

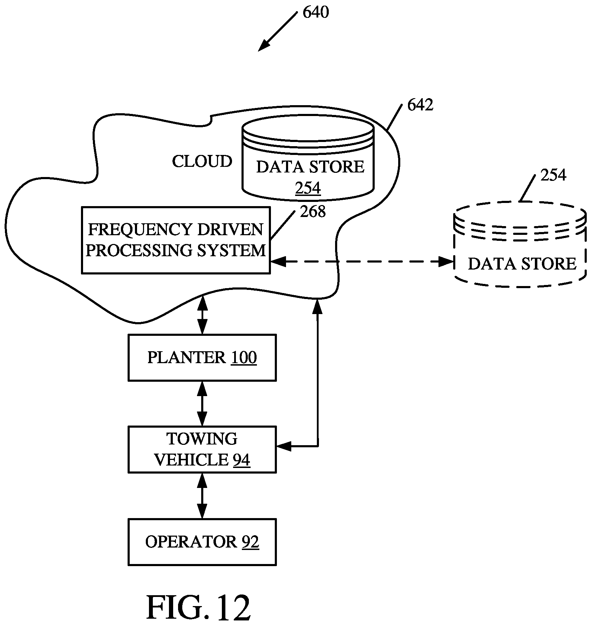

FIG. 12 is a block diagram of the architecture, shown in FIG. 1, except that it communicates with elements in a remote server architecture 640. In an example, remote server architecture 640 can provide computation, software, data access, and storage services that do not require end-user knowledge of the physical location or configuration of the system that delivers the services. In various embodiments, remote servers can deliver the services over a wide area network, such as the internet, using appropriate protocols. For instance, remote servers can deliver applications over a wide area network and they can be accessed through a web browser or any other computing component. Software or components shown in FIG. 8 as well as the corresponding data, can be stored on servers at a remote location. The computing resources in a remote server environment can be consolidated at a remote data center location or they can be dispersed. Remote server infrastructures can deliver services through shared data centers, even though they appear as a single point of access for the user. Thus, the components and functions described herein can be provided from a remote server at a remote location using a remote server architecture. Alternatively, they can be provided from a conventional server, or they can be installed on client devices directly, or in other ways.

FIG. 12 also depicts another example of a remote server architecture. FIG. 12 shows that it is also contemplated that some elements of FIGS. 1 and 8 can be disposed at remote server location 642 while others are not. By way of example, data store 254 can be disposed at a location separate from location 642 and accessed through the remote server at location 642. Regardless of where they are located, they can be accessed directly by system 113, through a network (either a wide area network or a local area network), they can be hosted at a remote site by a service, or they can be provided as a service, or accessed by a connection service that resides in a remote location. Also, the data can be stored in substantially any location and intermittently accessed by, or forwarded to, interested parties. For instance, physical carriers can be used instead of, or in addition to, electromagnetic wave carriers. In such an example, where cell coverage is poor or nonexistent, another mobile machine (such as a fuel truck) can have an automated information collection system. As the planter comes close to the fuel truck for fueling, the system automatically collects the information from the planter using any type of ad-hoc wireless connection. The collected information can then be forwarded to the main network as the fuel truck reaches a location where there is cellular coverage (or other wireless coverage). For instance, the fuel truck may enter a covered location when traveling to fuel other machines or when at a main fuel storage location. All of these architectures are contemplated herein. Further, the information can be stored on the planter until the planter enters a covered location. The planter, itself, can then send the information to the main network.

It will also be noted that the elements of FIGS. 1 and 8, or portions of them, can be disposed on a wide variety of different devices. Some of those devices include servers, desktop computers, laptop computers, tablet computers, or other mobile devices, such as palm top computers, cell phones, smart phones, multimedia players, personal digital assistants, etc.

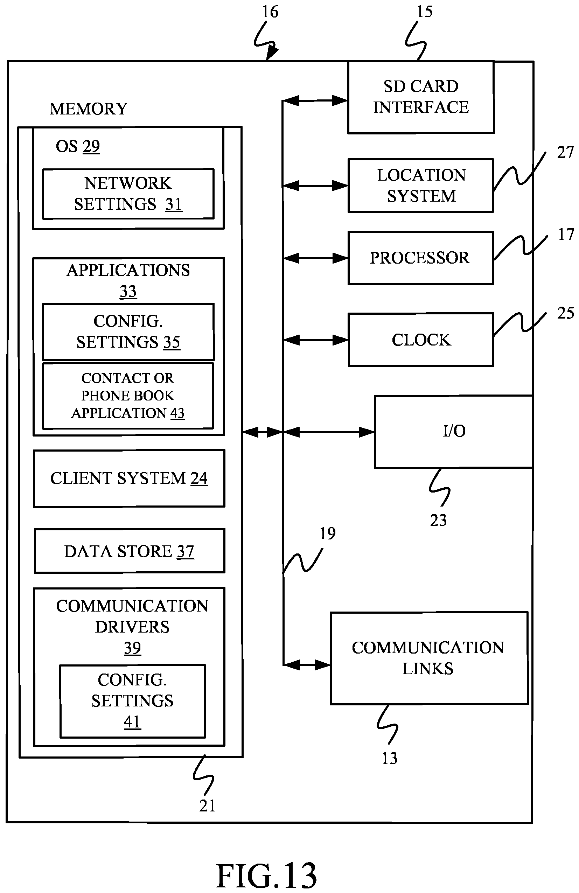

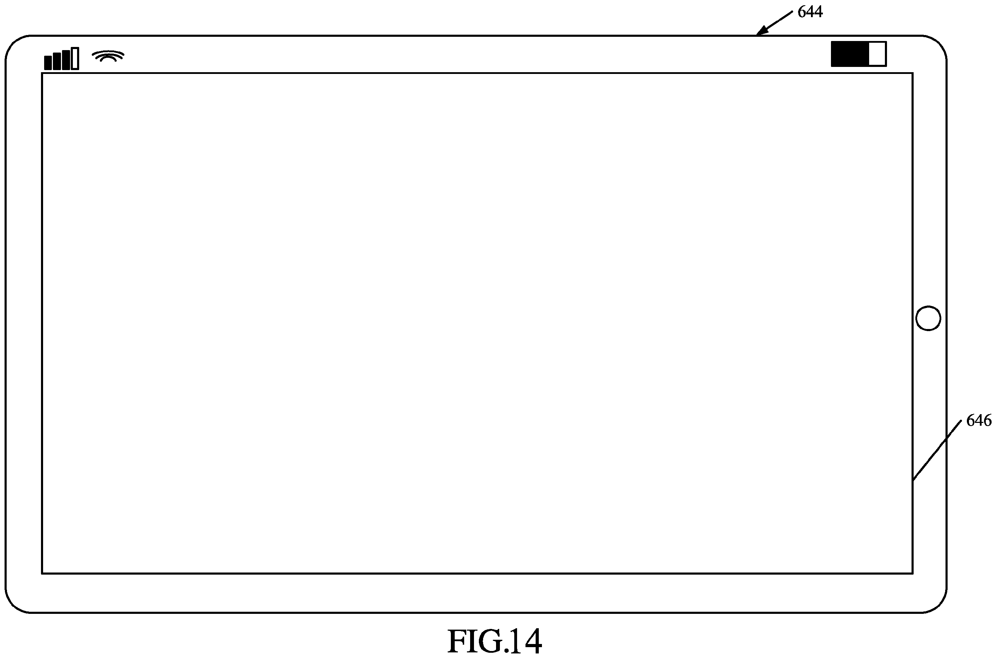

FIG. 13 is a simplified block diagram of one illustrative example of a handheld or mobile computing device that can be used as a user's or client's hand held device 16, in which the present system (or parts of it) can be deployed. For instance, a mobile device can be deployed in the operator compartment of towing vehicle 94 for use in generating, processing, or displaying the application data. FIGS. 14-15 are examples of handheld or mobile devices.

FIG. 13 provides a general block diagram of the components of a client device 16 that can run some components shown in FIGS. 1 and 8, that interacts with them, or both. In the device 16, a communications link 13 is provided that allows the handheld device to communicate with other computing devices and in some examples provides a channel for receiving information automatically, such as by scanning. Examples of communications link 13 include allowing communication though one or more communication protocols, such as wireless services used to provide cellular access to a network, as well as protocols that provide local wireless connections to networks.

In other examples, applications can be received on a removable Secure Digital (SD) card that is connected to an interface 15. Interface 15 and communication links 13 communicate with a processor 17 (which can also embody processors from previous FIGS.) along a bus 19 that is also connected to memory 21 and input/output (I/O) components 23, as well as clock 25 and location system 27.

I/O components 23, in one example, are provided to facilitate input and output operations. I/O components 23 for various examples of the device 16 can include input components such as buttons, touch sensors, optical sensors, microphones, touch screens, proximity sensors, accelerometers, orientation sensors and output components such as a display device, a speaker, and or a printer port. Other I/O components 23 can be used as well.

Clock 25 illustratively comprises a real time clock component that outputs a time and date. It can also, illustratively, provide timing functions for processor 17.

Location system 27 illustratively includes a component that outputs a current geographical location of device 16. This can include, for instance, a global positioning system (GPS) receiver, a LORAN system, a dead reckoning system, a cellular triangulation system, or other positioning system. It can also include, for example, mapping software or navigation software that generates desired maps, navigation routes and other geographic functions.

Memory 21 stores operating system 29, network settings 31, applications 33, application configuration settings 35, data store 37, communication drivers 39, and communication configuration settings 41. Memory 21 can include all types of tangible volatile and non-volatile computer-readable memory devices. It can also include computer storage media (described below). Memory 21 stores computer readable instructions that, when executed by processor 17, cause the processor to perform computer-implemented steps or functions according to the instructions. Processor 17 can be activated by other components to facilitate their functionality as well.

FIG. 14 shows one example in which device 16 is a tablet computer 644. In FIG. 14, computer 644 is shown with user interface display screen 646. Screen 646 can be a touch screen or a pen-enabled interface that receives inputs from a pen or stylus. It can also use an on-screen virtual keyboard. Of course, it might also be attached to a keyboard or other user input device through a suitable attachment mechanism, such as a wireless link or USB port, for instance. Computer 644 can also illustratively receive voice inputs as well.

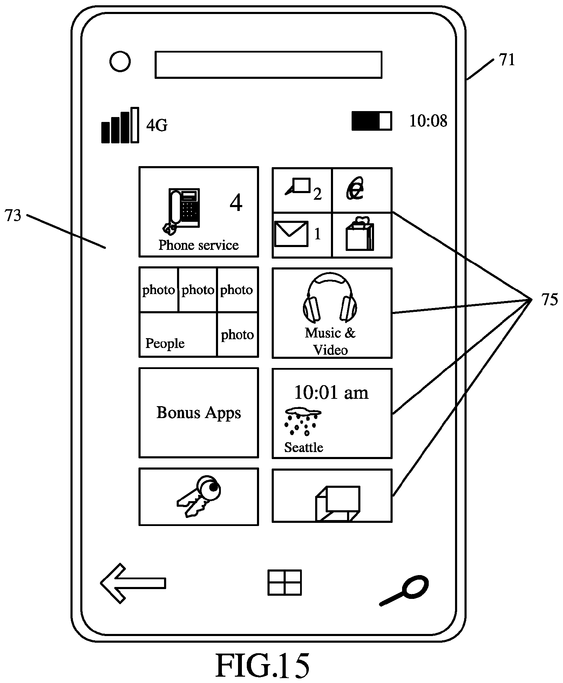

FIG. 15 shows that the device can be a smart phone 71. Smart phone 71 has a touch sensitive display 73 that displays icons or tiles or other user input mechanisms 75. Mechanisms 75 can be used by a user to run applications, make calls, perform data transfer operations, etc. In general, smart phone 71 is built on a mobile operating system and offers more advanced computing capability and connectivity than a feature phone.

Note that other forms of the devices 16 are possible.

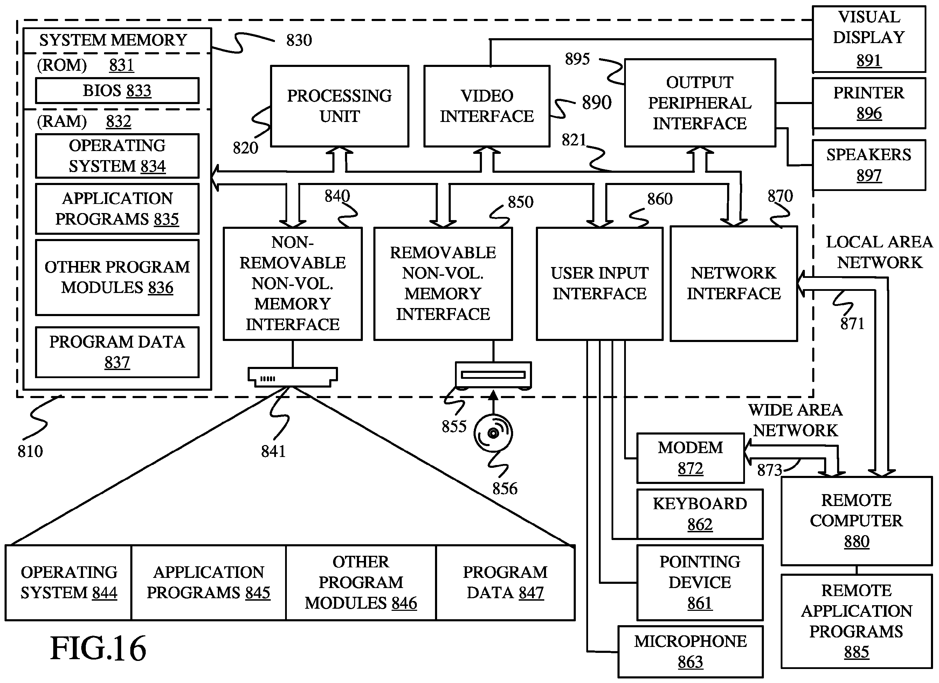

FIG. 16 is one example of a computing environment in which elements of FIGS. 1 and 8, or parts of it, (for example) can be deployed. With reference to FIG. 16, an example system for implementing some embodiments includes a general-purpose computing device in the form of a computer 810. Components of computer 810 may include, but are not limited to, a processing unit 820 (which can comprise processors from previous Figures), a system memory 830, and a system bus 821 that couples various system components including the system memory to the processing unit 820. The system bus 821 may be any of several types of bus structures including a memory bus or memory controller, a peripheral bus, and a local bus using any of a variety of bus architectures. Memory and programs described with respect to FIGS. 1 and 8 can be deployed in corresponding portions of FIG. 16.

Computer 810 typically includes a variety of computer readable media. Computer readable media can be any available media that can be accessed by computer 810 and includes both volatile and nonvolatile media, removable and non-removable media. By way of example, and not limitation, computer readable media may comprise computer storage media and communication media. Computer storage media is different from, and does not include, a modulated data signal or carrier wave. It includes hardware storage media including both volatile and nonvolatile, removable and non-removable media implemented in any method or technology for storage of information such as computer readable instructions, data structures, program modules or other data. Computer storage media includes, but is not limited to, RAM, ROM, EEPROM, flash memory or other memory technology, CD-ROM, digital versatile disks (DVD) or other optical disk storage, magnetic cassettes, magnetic tape, magnetic disk storage or other magnetic storage devices, or any other medium, which can be used to store the desired information and which can be accessed by computer 810. Communication media may embody computer readable instructions, data structures, program modules or other data in a transport mechanism and includes any information delivery media. The term "modulated data signal" means a signal that has one or more of its characteristics set or changed in such a manner as to encode information in the signal.

The system memory 830 includes computer storage media in the form of volatile and/or nonvolatile memory such as read only memory (ROM) 831 and random access memory (RAM) 832. A basic input/output system 833 (BIOS), containing the basic routines that help to transfer information between elements within computer 810, such as during start-up, is typically stored in ROM 831. RAM 832 typically contains data and/or program modules that are immediately accessible to and/or presently being operated on by processing unit 820. By way of example, and not limitation, FIG. 16 illustrates operating system 834, application programs 835, other program modules 836, and program data 837.

The computer 810 may also include other removable/non-removable volatile/nonvolatile computer storage media. By way of example only, FIG. 16 illustrates a hard disk drive 841 that reads from or writes to non-removable, nonvolatile magnetic media, an optical disk drive 855, and nonvolatile optical disk 856. The hard disk drive 841 is typically connected to the system bus 821 through a non-removable memory interface such as interface 840, and optical disk drive 855 are typically connected to the system bus 821 by a removable memory interface, such as interface 850.

Alternatively, or in addition, the functionality described herein can be performed, at least in part, by one or more hardware logic components. For example, and without limitation, illustrative types of hardware logic components that can be used include Field-programmable Gate Arrays (FPGAs), Application-specific Integrated Circuits (e.g., ASICs), Application-specific Standard Products (e.g., ASSPs), System-on-a-chip systems (SOCs), Complex Programmable Logic Devices (CPLDs), etc.

The drives and their associated computer storage media discussed above and illustrated in FIG. 15, provide storage of computer readable instructions, data structures, program modules and other data for the computer 810. In FIG. 16, for example, hard disk drive 841 is illustrated as storing operating system 844, application programs 845, other program modules 846, and program data 847. Note that these components can either be the same as or different from operating system 834, application programs 835, other program modules 836, and program data 837.

A user may enter commands and information into the computer 810 through input devices such as a keyboard 862, a microphone 863, and a pointing device 861, such as a mouse, trackball or touch pad. Other input devices (not shown) may include a joystick, game pad, satellite dish, scanner, or the like. These and other input devices are often connected to the processing unit 820 through a user input interface 860 that is coupled to the system bus, but may be connected by other interface and bus structures. A visual display 891 or other type of display device is also connected to the system bus 821 via an interface, such as a video interface 890. In addition to the monitor, computers may also include other peripheral output devices such as speakers 897 and printer 896, which may be connected through an output peripheral interface 895.

The computer 810 is operated in a networked environment using logical connections (such as a controller area network - CAN, local area network - LAN, or wide area network WAN) to one or more remote computers, such as a remote computer 880.

When used in a LAN networking environment, the computer 810 is connected to the LAN 871 through a network interface or adapter 870. When used in a WAN networking environment, the computer 810 typically includes a modem 872 or other means for establishing communications over the WAN 873, such as the Internet. In a networked environment, program modules may be stored in a remote memory storage device. FIG. 16 illustrates, for example, that remote application programs 885 can reside on remote computer 880.

It should also be noted that the different examples described herein can be combined in different ways. That is, parts of one or more examples can be combined with parts of one or more other examples. All of this is contemplated herein.

Although the subject matter has been described in language specific to structural features and/or methodological acts, it is to be understood that the subject matter defined in the appended claims is not necessarily limited to the specific features or acts described above. Rather, the specific features and acts described above are disclosed as example forms of implementing the claims.

As used herein, "e.g.," is utilized to non-exhaustively list examples and carries the same meaning as alternative illustrative phrases such as "including," "including, but not limited to," and "including without limitation." Unless otherwise limited or modified, lists with elements that are separated by conjunctive terms (e.g., "and") and that are also preceded by the phrase "one or more of" or "at least one of" indicate configurations or arrangements that potentially include individual elements of the list, or any combination thereof. For example, "at least one of A, B, and C" or "one or more of A, B, and C" indicates the possibilities of only A, only B, only C, or any combination of two or more of A, B, and C (e.g., A and B; B and C; A and C; or A, B, and C).

Those having ordinary skill in the art will recognize that terms such as "above," "below," "upward," "downward," "top," "bottom," etc., are used descriptively for the figures, and do not represent limitations on the scope of the disclosure, as defined by the appended claims. Furthermore, the teachings may be described herein in terms of functional and/or logical block components and/or various processing steps. It should be realized that such block components may be comprised of any number of hardware, software, and/or firmware components configured to perform the specified functions.

Terms of degree, such as "generally", "substantially" or "approximately" are understood by those of ordinary skill to refer to reasonable ranges outside of a given value or orientation, for example, general tolerances or positional relationships associated with manufacturing, assembly, and use of the described embodiments.

While the above describes example embodiments of the present disclosure, these descriptions should not be viewed in a limiting sense. Rather, other variations and modifications may be made without departing from the scope and spirit of the present disclosure as defined in the appended claims.

The foregoing description and examples have been set forth merely to illustrate the disclosure and are not intended as being limiting. Each of the disclosed aspects and embodiments of the present disclosure may be considered individually or in combination with other aspects, embodiments, and variations of the disclosure. In addition, unless otherwise specified, none of the steps of the methods of the present disclosure are confined to any particular order of performance. Modifications of the disclosed embodiments incorporating the spirit and substance of the disclosure may occur to persons skilled in the art and such modifications are within the scope of the present disclosure. Furthermore, all references cited herein are incorporated by reference in their entirety.

Terms of orientation used herein, such as "top," "bottom," "horizontal," "vertical," "longitudinal," "lateral," and "end" are used in the context of the illustrated embodiment. However, the present disclosure should not be limited to the illustrated orientation. Indeed, other orientations are possible and are within the scope of this disclosure. Terms relating to circular shapes as used herein, such as diameter or radius, should be understood not to require perfect circular structures, but rather should be applied to any suitable structure with a cross-sectional region that can be measured from side-to-side. Terms relating to shapes generally, such as "circular" or "cylindrical" or "semi-circular" or "semi-cylindrical" or any related or similar terms, are not required to conform strictly to the mathematical definitions of circles or cylinders or other structures but can encompass structures that are reasonably close approximations.

Conditional language used herein, such as, among others, "can," "might," "may," "e.g.," and the like, unless specifically stated otherwise, or otherwise understood within the context as used, is generally intended to convey that some embodiments include, while other embodiments do not include, certain features, elements, and/or states. Thus, such conditional language is not generally intended to imply that features, elements, blocks, and/or states are in any way required for one or more embodiments or that one or more embodiments necessarily include logic for deciding, with or without author input or prompting, whether these features, elements and/or states are included or are to be performed in any particular embodiment.

Conjunctive language, such as the phrase "at least one of X, Y, and Z," unless specifically stated otherwise, is otherwise understood with the context as used in general to convey that an item, term, etc. may be either X, Y, or Z. Thus, such conjunctive language is not generally intended to imply that certain embodiments require the presence of at least one of X, at least one of Y, and at least one of Z.

The terms "approximately," "about," and "substantially" as used herein represent an amount close to the stated amount that still performs a desired function or achieves a desired result. For example, in some embodiments, as the context may dictate, the terms "approximately", "about", and "substantially" may refer to an amount that is within less than or equal to 10% of the stated amount. The term "generally" as used herein represents a value, amount, or characteristic that predominantly includes or tends toward a particular value, amount, or characteristic. As an example, in certain embodiments, as the context may dictate, the term "generally parallel" can refer to something that departs from exactly parallel by less than or equal to 20 degrees.

Unless otherwise explicitly stated, articles such as "a" or "an" should generally be interpreted to include one or more described items. Accordingly, phrases such as "a device configured to" are intended to include one or more recited devices. Such one or more recited devices can be collectively configured to carry out the stated recitations. For example, "a processor configured to carry out recitations A, B, and C" can include a first processor configured to carry out recitation A working in conjunction with a second processor configured to carry out recitations B and C.

The terms "comprising," "including," "having," and the like are synonymous and are used inclusively, in an open-ended fashion, and do not exclude additional elements, features, acts, operations, and so forth. Likewise, the terms "some," "certain," and the like are synonymous and are used in an open-ended fashion. Also, the term "or" is used in its inclusive sense (and not in its exclusive sense) so that when used, for example, to connect a list of elements, the term "or" means one, some, or all of the elements in the list.

Overall, the language of the claims is to be interpreted broadly based on the language employed in the claims. The language of the claims is not to be limited to the non-exclusive embodiments and examples that are illustrated and described in this disclosure, or that are discussed during the prosecution of the application.

Although systems and methods for controlling a planting machine have been disclosed in the context of certain embodiments and examples, this disclosure extends beyond the specifically disclosed embodiments to other alternative embodiments and/or uses of the embodiments and certain modifications and equivalents thereof. Various features and aspects of the disclosed embodiments can be combined with or substituted for one another in order to form varying modes of systems and methods for controlling a planting machine. The scope of this disclosure should not be limited by the particular disclosed embodiments described herein.

Certain features that are described in this disclosure in the context of separate implementations can be implemented in combination in a single implementation. Conversely, various features that are described in the context of a single implementation can be implemented in multiple implementations separately or in any suitable subcombination. Although features may be described herein as acting in certain combinations, one or more features from a claimed combination can, in some cases, be excised from the combination, and the combination may be claimed as any subcombination or variation of any subcombination.

While the methods and devices described herein may be susceptible to various modifications and alternative forms, specific examples thereof have been shown in the drawings and are herein described in detail. It should be understood, however, that the invention is not to be limited to the particular forms or methods disclosed, but, to the contrary, the invention is to cover all modifications, equivalents, and alternatives falling within the spirit and scope of the various embodiments described and the appended claims. Further, the disclosure herein of any particular feature, aspect, method, property, characteristic, quality, attribute, element, or the like in connection with an embodiment can be used in all other embodiments set forth herein. Any methods disclosed herein need not be performed in the order recited. Depending on the embodiment, one or more acts, events, or functions of any of the algorithms, methods, or processes described herein can be performed in a different sequence, can be added, merged, or left out altogether (e.g., not all described acts or events are necessary for the practice of the algorithm). In some embodiments, acts or events can be performed concurrently, e.g., through multi-threaded processing, interrupt processing, or multiple processors or processor cores or on other parallel architectures, rather than sequentially. Further, no element, feature, block, or step, or group of elements, features, blocks, or steps, are necessary or indispensable to each embodiment. Additionally, all possible combinations, subcombinations, and rearrangements of systems, methods, features, elements, modules, blocks, and so forth are within the scope of this disclosure. The use of sequential, or time-ordered language, such as "then," "next," "after," "subsequently," and the like, unless specifically stated otherwise, or otherwise understood within the context as used, is generally intended to facilitate the flow of the text and is not intended to limit the sequence of operations performed. Thus, some embodiments may be performed using the sequence of operations described herein, while other embodiments may be performed following a different sequence of operations.

Moreover, while operations may be depicted in the drawings or described in the specification in a particular order, such operations need not be performed in the particular order shown or in sequential order, and all operations need not be performed, to achieve the desirable results. Other operations that are not depicted or described can be incorporated in the example methods and processes. For example, one or more additional operations can be performed before, after, simultaneously, or between any of the described operations. Further, the operations may be rearranged or reordered in other implementations. Also, the separation of various system components in the implementations described herein should not be understood as requiring such separation in all implementations, and it should be understood that the described components and systems can generally be integrated together in a single product or packaged into multiple products. Additionally, other implementations are within the scope of this disclosure.

Some embodiments have been described in connection with the accompanying figures. Certain figures are drawn and/or shown to scale, but such scale should not be limiting, since dimensions and proportions other than what are shown are contemplated and are within the scope of the embodiments disclosed herein. Distances, angles, etc. are merely illustrative and do not necessarily bear an exact relationship to actual dimensions and layout of the devices illustrated. Components can be added, removed, and/or rearranged. Further, the disclosure herein of any particular feature, aspect, method, property, characteristic, quality, attribute, element, or the like in connection with various embodiments can be used in all other embodiments set forth herein. Additionally, any methods described herein may be practiced using any device suitable for performing the recited steps.

The methods disclosed herein may include certain actions taken by a practitioner; however, the methods can also include any third-party instruction of those actions, either expressly or by implication. For example, actions such as "positioning an electrode" include "instructing positioning of an electrode."

The ranges disclosed herein also encompass any and all overlap, subranges, and combinations thereof. Language such as "up to," "at least," "greater than," "less than," "between," and the like includes the number recited. Numbers preceded by a term such as "about" or "approximately" include the recited numbers and should be interpreted based on the circumstances (e.g., as accurate as reasonably possible under the circumstances, for example ±5%, ±10%, ±15%, etc.). For example, "about 1 V" includes "1 V." Phrases preceded by a term such as "substantially" include the recited phrase and should be interpreted based on the circumstances (e.g., as much as reasonably possible under the circumstances). For example, "substantially perpendicular" includes "perpendicular." Unless stated otherwise, all measurements are at standard conditions including temperature and pressure.

In summary, various embodiments and examples of systems and methods for controlling a planting machine has been disclosed. Although the systems and methods for controlling a planting machine have been disclosed in the context of those embodiments and examples, this disclosure extends beyond the specifically disclosed embodiments to other alternative embodiments and/or other uses of the embodiments, as well as to certain modifications and equivalents thereof. This disclosure expressly contemplates that various features and aspects of the disclosed embodiments can be combined with, or substituted for, one another. Thus, the scope of this disclosure should not be limited by the particular disclosed embodiments described herein but should be determined only by a fair reading of the claims that follow.
Embodiments of the invention can be described with reference to the following numbered clauses, with preferred features laid out in dependent clauses:
1. A planting machine comprising:
   a processing system;
   a moisture measurement device in communication with the processing system;
   a seed delivery system in communication with the processing system;
   a material dispersion system in communication with the processing system,
   wherein the processing system is configured to:
      receive one or more soil moisture measurement signals from the soil moisture measurement device;
      determine one or more material dispersion parameters based at least in part on a moisture measurement signal; and
      transmit a control signal to one or more of the seed delivery system and the material dispersion system to disburse a desired amount of material with a commodity based at least in part on the determined material dispersion parameters.
2. The planting machine of clause 1, wherein the one or more material dispersion parameters includes whether to disperse the material onto the commodity or adjacent the commodity.
3. The planting machine of clause 1 or clause 2, wherein the one or more material dispersion parameters includes an amount of material to disburse.
4. The planting machine of any one of the preceding clauses, wherein the processing system is further configured to receive soil-type data, and wherein the one or more material dispersion parameters is based at least in part on the soil-type data.
5. The planting machine of any one of the preceding clauses, further comprising:
   determining a position of the planting machine;
   determining associated soil parameters;
   determining soil identification data based at least in part on the position of the planting machine and the associated soil parameters.
6. The planting machine of clause 5 wherein the processing system is further configured to determine one or more material dispersion parameters based at least in part on soil-type data.
7. The planting machine of any one of the preceding clauses wherein the processing system is further configured to communicate moisture measurements from the moisture measurement device to a central server.
8. The planting machine of any one of the preceding clauses, wherein the moisture measurement device comprises non-contact moisture measurement device.
9. The planting machine of any one of the preceding clauses, wherein the moisture measurement device comprises an electromagnetic wave-based moisture measurement device.
10. The planting machine of any one of the preceding clauses, wherein the moisture measurement device comprises an insertable moisture measurement device.
11. A method of controlling a planting machine comprising:
   receiving one or more soil moisture measurement signals from the soil moisture measurement device;
   determining one or more material dispersion parameters based at least in part on one or more of the moisture measurement signals; and
   transmitting a control signal to the material dispersion system to disburse a desired amount of material with the commodity based at least in part on the determined amount of material.
12. The method of clause 11, wherein the one or more material dispersion parameters includes, whether to disburse the material directly onto the commodity or adjacent the commodity.
13. The method of clause 11 or clause 12, wherein the one or more material dispersion parameters includes an amount of material to disburse.
14. The method of any one of clauses 11 to 13, further comprising receiving soil-composition data, and wherein the one or more material dispersion parameters is based at least in part on the soil-type data.
15. The method of clause 14, wherein the soil-composition data comprises pre-measured data.
16. The method of any one of clauses 11 to 15, further comprising:
   determining a position of the planting machine;
   determining associated soil parameters;
   determining soil identification data based at least in part on the position of the planting machine and the associated soil parameters.
17. The method of clause 16, wherein the position of the planting machine is based at least in part on global positioning data.
18. The method of clause 16 or clause 17, further comprising determining one or more material dispersion parameters based at least in part on soil identification data.
19. The method of any one of clauses 16 to 18, further comprising communicating soil identification data to a central server.
20. The method of any one of clauses 11 to 19, wherein the one or more soil moisture measurement signals are based at least in part on electromagnetic wave measurement data.

## Claims

1. A planting machine comprising:
a processing system;
a moisture measurement device in communication with the processing system;
a seed delivery system in communication with the processing system;
a material dispersion system in communication with the processing system,
wherein the processing system is configured to:
receive one or more soil moisture measurement signals from the soil moisture measurement device;
determine one or more material dispersion parameters based at least in part on a moisture measurement signal; and
transmit a control signal to one or more of the seed delivery system and the material dispersion system to disburse a desired amount of material with a commodity based at least in part on the determined material dispersion parameters.

2. The planting machine of claim 1, wherein the one or more material dispersion parameters includes whether to disperse the material onto the commodity or adjacent the commodity.

3. The planting machine of claim 1 or claim 2, wherein the one or more material dispersion parameters includes an amount of material to disburse.

4. The planting machine of any one of the preceding claims, wherein the processing system is further configured to receive soil-type data, and wherein the one or more material dispersion parameters is based at least in part on the soil-type data.

5. The planting machine of any one of the preceding claims, further comprising:
determining a position of the planting machine;
determining associated soil parameters;
determining soil identification data based at least in part on the position of the planting machine and the associated soil parameters.

6. The planting machine of any one of the preceding claims, wherein the moisture measurement device comprises non-contact moisture measurement device.

7. The planting machine of any one of the preceding claims, wherein the moisture measurement device comprises an insertable moisture measurement device.

8. A method of controlling a planting machine comprising:
receiving one or more soil moisture measurement signals from the soil moisture measurement device;
determining one or more material dispersion parameters based at least in part on one or more of the moisture measurement signals; and
transmitting a control signal to the material dispersion system to disburse a desired amount of material with the commodity based at least in part on the determined amount of material.

9. The method of claim 8, wherein the one or more material dispersion parameters includes, whether to disburse the material directly onto the commodity or adjacent the commodity.

10. The method of claim 8 or claim 9, wherein the one or more material dispersion parameters includes an amount of material to disburse.

11. The method of any one of claims 8 to 10, further comprising receiving soil-composition data, and wherein the one or more material dispersion parameters is based at least in part on the soil-type data.

12. The method of any one of claims 8 to 11, further comprising:
determining a position of the planting machine;
determining associated soil parameters;
determining soil identification data based at least in part on the position of the planting machine and the associated soil parameters.

13. The method of claim 12, wherein the position of the planting machine is based at least in part on global positioning data.

14. The method of claim 12 or claim 13, further comprising determining one or more material dispersion parameters based at least in part on soil identification data.

15. The method of any one of claims 12 to 14, further comprising communicating soil identification data to a central server
